# EUROPEAN PATENT SPECIFICATION

(11) **EP 4 561 985 B1**
(45) Date of publication and mention of the grant of the patent: **03.12.2025**
(21) Application number: 23754361.6
(22) Date of filing: 28.07.2023
(51) Int. Cl.: C07C 237/32, C08G 59/54, C08G 59/62

(54) **AMIDO-AMINE HARDENER**
AMIDOAMINHÄRTER
DURCISSEUR AMIDO-AMINE

(30) Priority: 28.07.2022 GB 202211052
(43) Date of publication of application: 04.06.2025
(73) Proprietor: Jones, Paul, Wrightington WN6 9PS (GB)
(72) Inventor: Jones, Paul, Wrightington WN6 9PS (GB)
(74) Representative: Mathys & Squire
(86) International application number: PCT/GB2023/052009
(87) International publication number: WO 2024/023531

(56) References cited:
- EP-A1- 0 779 311
- GB-A- 1 309 454
- US-A1- 2017 355 695
- DATABASE REGISTRY [online] Chemical Abstracts Service, Columbus, OH, US; 8 January 2014 (2014-01-08), ANON.: "2-Furancarboxamide, N-[(1R,3R)-3-aminocyclopentyl]-4-[(diethylamino)methyl]-5-ethyl-, rel-", XP093096883, retrieved from STN Database accession no. 1513928-56-7
- DATABASE REGISTRY [online] Chemical Abstracts Service, Columbus, OH, US; 15 November 2016 (2016-11-15), ANON.: "2-Thiophenecarboxamide, N-(3-aminobicyclo[3.3.1]non-9-yl)-3-[(dimethylamino)methyl]-, hydrochloride (1:2)", XP093096884, retrieved from STN Database accession no. 2031502-63-1
- DATABASE REGISTRY [online] Chemical Abstracts Service, Columbus, OH, US; 25 November 2016 (2016-11-25), ANON.: "2-Thiophenecarboxamide, 3-[(dimethylamino)methyl]-N-[2-(methylamino)propyl]-, hydrochloride (1:2)", XP093096887, retrieved from STN Database accession no. 2038006-91-4
- DATABASE REGISTRY [online] Chemical Abstracts Service, Columbus, OH, US; 29 November 2016 (2016-11-29), ANON.: "2-Thiophenecarboxamide, 3-[(dimethylamino)methyl]-N-methyl-N-[2-(methylamino)ethyl]-, hydrochloride (1:2)", XP093096890, retrieved from STN Database accession no. 2040692-48-4
- DATABASE REGISTRY [online] Chemical Abstracts Service, Columbus, OH, US; 7 March 2019 (2019-03-07), ANON.: "2-Furancarboxamide, N-(3-aminopropyl)-5-[(methylamino)methyl]-", XP093096891, retrieved from STN Database accession no. 2280814-40-4
- DATABASE REGISTRY [online] Chemical Abstracts Service, Columbus, OH, US; 15 June 2020 (2020-06-15), ANON.: "2-Thiophenecarboxamide, 3-[(dimethylamino)methyl]-N-[2-methyl-2-[(1-phenylethyl)amino]propyl]-", XP093096893, retrieved from STN Database accession no. 2424932-11-4
- DATABASE REGISTRY [online] Chemical Abstracts Service, Columbus, OH, US; 18 June 2020 (2020-06-18), ANON.: "2-Thiophenecarboxamide, N-(3-amino-1-methylbutyl)-3-[(dimethylamino)methyl]-", XP093096896, retrieved from STN Database accession no. 2428319-43-9

## Description

The invention relates to a class of amido-amines useful as curatives. In particular, the invention is directed to compounds comprising at least one amide group and at least one Mannich base, polymeric compounds comprising a repeating unit having at least one amide group and at least one Mannich base, a resin curative composition comprising such compounds, processes for preparing them and uses thereof.

### BACKGROUND OF THE INVENTION

There is an array of crosslinking agents available for epoxy functional materials, but amines and products derived from amines offer the greatest versatility for curing epoxy resins. Collectively these materials offer the means of formulating systems that can provide the potential for curing in thin films and/or mass at a broad spectrum of temperatures. Polyamides and phenolic derived Mannich base curing agents have been used extensively but due to the inability of the polyamides to low temperature cure, and the regulatory and toxicity issues associated with free phenol, the scope of use and availability of these materials has substantially reduced.

Many commercial curing agent formulations are based on amines such as aliphatic, cyclo-aliphatic aryl-aliphatic and to a lesser extent aromatic amines or combinations thereof. These amines are generally modified in order to enhance the processing and/or performance aspects, to improve the active hydrogen equivalent weight and combining ratio with epoxy resins or to reduce the toxicity of the amine.

Amido-amines and polyamides are examples of modified curing agents that are used in ambient cured systems above 10°C and Mannich bases are examples of an amine modification to offer enhanced properties especially with regard to improved compatibility with epoxy resins, the alteration of the reactivity and cure speed, as well as the degree of cure at low temperatures (e.g. <10°C). They can also enhance resistance to carbamation whilst curing and aside from use in their own right, they can be used to boost the performance of amido-amine and polyamide curing agents.

Amido-amines and polyamides may be the reaction product of a fatty acid and/or dimer fatty acids (for example, mixtures of mono-di- and trifunctional acids) with an amine. An example of a commercially available polyamide curative is Versamid 140 (RTM).

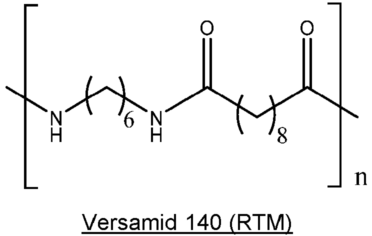

Mannich bases are examples of modified amines which offer enhanced properties, especially with regard to improved compatibility with epoxy resins, optimisation of cure speed and degree of cure, as well as resistance to carbamation. Commercially available Mannich bases include phenolic derived compounds that are the reaction product of an aldehyde (generally formaldehyde), a phenolic compound, or a substituted derivative thereof, and an amine. An example structure of a phenolic Mannich base is shown below:

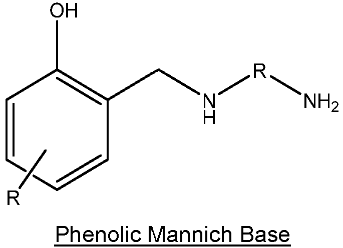

EP0779311 describes a Mannich base prepared by reacting (i) butyraldehyde, (ii) a phenolic compound, and (iii) a primary or a secondary polyamine. The use of butyraldehyde, rather than formaldehyde or paraformaldehyde, is reported to lower viscosity in the Mannich base products, which can be advantageous when used in epoxy curative applications.

However, the molecular weight, polydispersity and residual free phenol monomer levels has led to the reduction in availability and a decline in popularity of this class of materials. The conventional Mannich base with residual free phenol carries both acute toxicity labelling and a chronic health hazard. The removal of several of the Hazard statements associated with free monomeric phenol and substituted phenols, is now mandated by some organisations, e.g. REACH (Registration, Evaluation, Authorisation and restriction of Chemicals). There remains a need for alternative Mannich bases that offer high process performance, broad compatibility with epoxy resins, and avoid the toxicity and environmental issues associated with known amine-derived epoxy resin curatives.

The present invention relates to a class of amide Mannich bases. An aromatic or heteroaromatic acid group or analogue thereof, such as salicylic acid and analogues thereof, undergoes amidation to form an amide, as well as the Mannich reaction to form a Mannich base. The amide Mannich bases of the present invention are formed using polyamines having at least two nucleophilic amines, for the amidation and preferably for the Mannich reaction, resulting in an amide Mannich base with at least one nucleophilic amine group side chain suitable for reaction with the epoxide group of an epoxy resin.

Amide Mannich bases, such as salicylamide Mannich bases for example, have been reported in the literature for use in pharmaceuticals and textile fibres. However, amide Mannich bases having the required structure for use as a curative are hitherto unknown.

Salicylamide Mannich bases have been investigated as drug-like scaffolds in Dank, C. et al. Hybrids of Salicylalkylamides and Mannich Bases: Control of the Amide Conformation by Hydrogen Bonding in Solution and in the Solid State. Molecules. 20, 1686-1711 (2015). However, Dank, C. *et al.* discloses only alkyl side chain on the salicylamide and Mannich base groups.

US 2,385,940 also discloses salicylamide Mannich bases for use in textile fabrics to aid water repellence. However, this purpose requires hydrophobic side chains which precludes the use of polyamines in the Mannich and/or amidation reactions.

### SUMMARY OF THE INVENTION

In one aspect, the present invention provides a compound of formula (2): 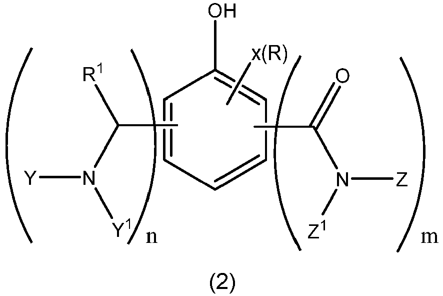 wherein:
each R group is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkyloxy, C₁ to C₂₀ alkylamino, -OH, -OR², -NH₂, -NHR², - NR²₂, -C(O)OH, -C(O)OR², -C(O)NH₂, -C(O)NHR², -C(O)NR²₂, -O(CO)H, -O(CO)R², -NH(CO)H, -NH(CO)R², -NR²(CO)H, -NR²(CO)R², -SH, -SR², -SO₂H, -SO₂R², - SO₃R², -SO₃H, -SiR²₃, -NO₂, -CN, -F, -Cl, -Br, and -I;
each R¹ group is independently selected from: -H, C₁ to C₁₀ alkyl, C₄ to C₁₀ haloalkyl, C₃ to C₁₂ cycloalkyl, C₂ to C₁₀ alkenyl, C₂ to C₁₀ alkynyl, C₆ to C₁₂ aryl, and C₃ to C₁₂ heteroaryl;
each R² is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkoxy, and C₁ to C₂₀ alkylamino;
Y is independently selected from a C₁ to C₂₅₀ hydrocarbyl group;
Y¹ is independently selected from hydrogen or a C₁ to C₂₅₀ hydrocarbyl group;
Z is independently selected from a C₂ to C₂₅₀ hydrocarbyl group comprising at least one primary or secondary amine;
Z¹ is independently selected from hydrogen or a C₁ to C₂₅₀ hydrocarbyl group;
n is independently 1, 2 or 3;
m is independently 1 or 2;
x is independently 0 to 3;
wherein n + m + x = 2 to 5; and
wherein the term hydrocarbyl refers to a monovalent group comprising a major proportion (i.e., more than 50 %) of hydrogen and carbon atoms, preferably consisting exclusively of hydrogen and carbon atoms.

In another aspect, the present invention provides a polymeric or co-polymeric compound comprising a repeating unit of formula (4): 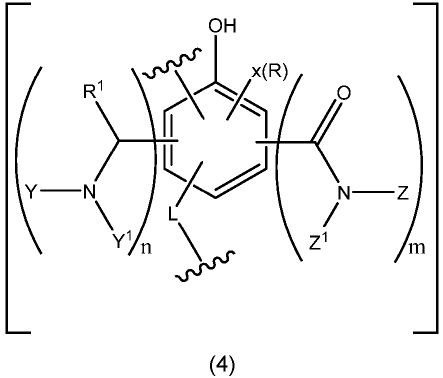 wherein:
R is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkyloxy, C₁ to C₂₀ alkylamino, -OH, -OR², -NH₂, -NHR², -NR²₂, -C(O)OH, -C(O)OR², -C(O)NH₂, -C(O)NHR², -C(O)NR²₂, -O(CO)H, -O(CO)R², -NH(CO)H, -NH(CO)R², -NR²(CO)H, -NR²(CO)R², -SH, -SR², -SO₂H, -SO₂R², -S0_{3R}², -SO₃H, -SiR²₃, -NO₂, -CN, -F, -Cl, -Br, and -I;
each R¹ group is independently selected from: -H, C₁ to C₁₀ alkyl, C₁ to C₁₀ haloalkyl, C₃ to C₁₂ cycloalkyl, C₂ to C₁₀ alkenyl, C₂ to C₁₀ alkynyl, C₆ to C₁₂ aryl, and C₃ to C₁₂ heteroaryl;
each R² is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkoxy, and C₁ to C₂₀ alkylamino;
Y is independently selected from a C₁ to C₂₅₀ hydrocarbyl group;
Y¹ is independently selected from hydrogen or a C₁ to C₂₅₀ hydrocarbyl group;
Z is independently selected from a C₂ to C₂₅₀ hydrocarbyl group comprising at least one primary or secondary amine;
Z¹ is independently either hydrogen or a C₁ to C₂₅₀ hydrocarbyl group; and
L is a linking group which connects the repeating unit to a single adjacent repeating unit of the polymer or co-polymer, and is selected from -CR⁹R¹⁰-;
each 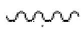 represents the attachment point between the linking group, L, of one repeating unit and a single adjacent repeating unit;
each R⁹ group is independently selected from: -H, C₁ to C₁₀ alkyl, C₁ to C₁₀ haloalkyl, C₃ to C₁₂ cycloalkyl, C₂ to C₁₀ alkenyl, C₂ to C₁₀ alkynyl, C₆ to C₁₂ aryl, C₃ to C₁₂ heteroaryl, and -C(O)H;
each R¹⁰ group is independently selected from: -H, C₁ to C₁₀ alkyl, C₁ to C₁₀ haloalkyl, C₃ to C₁₂ cycloalkyl, C₂ to C₁₀ alkenyl, C₂ to C₁₀ alkynyl, C₆ to C₁₂ aryl, and C₃ to C₁₂ heteroaryl;
or R⁹ and R¹⁰ are taken together to form a C₃ to C₁₂ cycloalkyl;
n is independently 1 or 2;
m is independently 1 or 2;
x is independently 0 or 1;
with the proviso that n + m + x = 2 or 3; and
wherein the term hydrocarbyl refers to a monovalent group comprising a major proportion (i.e., more than 50 %) of hydrogen and carbon atoms, preferably consisting exclusively of hydrogen and carbon atoms.

In another aspect, the present invention provides a polymeric or co-polymeric compound comprising a repeating unit of formula (5): 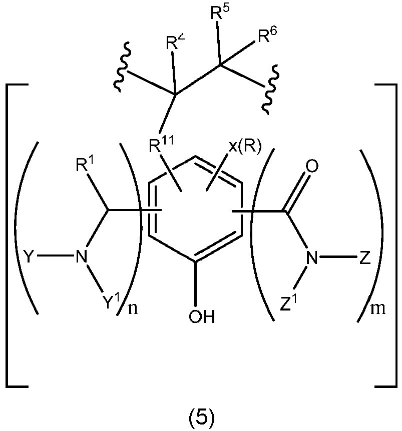 wherein:
R is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkyloxy, C₁ to C₂₀ alkylamino, -OH, -OR², -NH₂, -NHR², -NR²₂, - C(O)OH, -C(O)OR², -C(O)NH₂, -C(O)NHR², -C(O)NR²₂, -O(CO)H, -O(CO)R², - NH(CO)H, -NH(CO)R², -NR²(CO)H, -NR²(CO)R², -SH, -SR², -SO₂H, -SO₂R², - SO₃R², -SO₃H, -SiR²₃, -NO₂, -CN, -F, -Cl, -Br, and -I;
each R¹ group is independently selected from: -H, C₁ to C₁₀ alkyl, C₁ to C₁₀ haloalkyl, C₃ to C₁₂ cycloalkyl, C₂ to C₁₀ alkenyl, C₂ to C₁₀ alkynyl, C₆ to C₁₂ aryl, and C₃ to C₁₂ heteroaryl;
each R² is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkoxy, and C₁ to C₂₀ alkylamino;
Y is independently a C₁ to C₂₅₀ hydrocarbyl group;
Y¹ is independently either hydrogen or a C₁ to C₂₅₀ hydrocarbyl group;
Z is independently a C₂ to C₂₅₀ hydrocarbyl group comprising at least one primary or secondary amine;
Z¹ is independently either hydrogen or a C₁ to C₂₅₀ hydrocarbyl group; and R⁴, R⁵ and R⁶ are independently selected from: -H, C₁ to C₁₀ alkyl, -F, -Cl, -Br, -I, -OH, -OR¹, -CN, -C(O)OH, -C(O)OR¹, -C(O)NH₂, -C(O)NHR¹, -C(O)NR¹₂, -O(CO)H, or -O(CO)R¹;
each 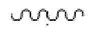 represents an attachment point between one repeating unit and a single adjacent repeating unit;
R¹¹ is either a direct bond or -OC(O)-;
n is independently 1, 2 or 3;
m is independently 1, 2 or 3;
x is independently 0 to 2;

with the proviso that n + m + x = 2 to 4; and
wherein the term hydrocarbyl refers to a monovalent group comprising a major proportion (i.e., more than 50 %) of hydrogen and carbon atoms, preferably consisting exclusively of hydrogen and carbon atoms.

In yet another aspect, the present invention provides a method of preparing an aromatic or heteroaromatic Mannich base for use as an epoxy resin curative and/or accelerator, said method comprising:
i) reacting an optionally substituted aromatic or heteroaromatic acid, or ester thereof, selected from hydroxy benzoic acid, hydroxypyridine carboxylic acid, pyrrole carboxylic acid, thiophene carboxylic acid, and furan carboxylic acid, or an ester thereof, with a first polyamine having at least two nucleophilic amines, to form an aromatic or heteroaromatic amide;
ii) performing a Mannich reaction using the aromatic or heteroaromatic amide, an aldehyde, and a second amine having at least one nucleophilic amine.

In yet another aspect, the present invention provides a method of preparing a polymeric hydroxy benzamide Mannich base for use as an epoxy resin curative and/or accelerator, said method comprising:
i) performing a polycondensation reaction with an optionally substituted hydroxy benzoic acid, or ester thereof, and a first aldehyde, ketone or hexamine to give a polymeric hydroxy benzoic acid, or ester thereof;
ii) reacting the polymeric hydroxy benzoic acid, or ester thereof, with a first polyamine having at least two nucleophilic amines, to give a polymeric hydroxy benzamide;
iii) performing a Mannich reaction using the polymeric hydroxy benzamide, a second aldehyde, and a second amine having at least one nucleophilic amine.

In yet another aspect, the present invention provides a method of preparing a polymeric hydroxy benzamide Mannich base for use as an epoxy resin curative and/or accelerator, said method comprising:
i) performing a vinyl polymerisation reaction with optionally substituted and alkenyl substituted hydroxy benzoic acid, or ester thereof, to give a polymeric hydroxy benzoic acid, or ester thereof;
ii) reacting the polymeric hydroxy benzoic acid, or ester thereof, with a first polyamine having at least two nucleophilic amines, to give a polymeric hydroxy benzamide;
iii) performing a Mannich reaction using the polymeric hydroxy benzamide, an aldehyde, and a second amine having at least one nucleophilic amine.

In yet another aspect, the present invention provides an epoxy resin curative composition comprising a compound as described herein.

In yet another aspect, the present invention provides a method for preparing a cured epoxy resin, said method comprising:
a) contacting an epoxy resin with a compound as described herein; and
b) forming a cured epoxy resin, preferably wherein the epoxy resin is selected from glycidyl amines, epoxidized novolacs and bisphenols (A or F) or halogenated analogues thereof.

In yet another aspect, the present invention provides a cured epoxy resin prepared, or preparable, by the methods described herein.

In a final aspect, the present invention provides a use of a compound as described herein for causing crosslinking of an epoxy resin.

### BRIEF DESCRIPTION OF THE FIGURES

Figure 1 demonstrates the viscosity vs dilution properties of the salicylamide Mannich base of Example 4 with benzyl alcohol; and
Figure 2 demonstrates the dry time and cure time of the salicylamide Mannich base of Example 4 diluted with xylene as compared to an industry standard polyamide.

### DETAILED DESCRIPTION

### Definition of terms

For the purposes of the present invention, the following terms as used herein shall, unless otherwise indicated, be understood to have the following meanings. Other terms that are not specifically defined below are to be understood as their normal meaning in the art.

The term "hydrocarbyl" as used herein, refers to a monovalent or divalent group, comprising a major proportion (i.e., more than 50 %) of hydrogen and carbon atoms, preferably consisting exclusively of hydrogen and carbon atoms. The hydrocarbyl group may be aromatic, saturated aliphatic or unsaturated aliphatic. The hydrocarbyl group may be entirely aliphatic or a combination of aliphatic and aromatic portions. In some examples, the hydrocarbyl group includes a branched aliphatic chain which is substituted by one or more aromatic groups. Examples of hydrocarbyl groups therefore include acyclic groups, as well as groups that combine one or more acyclic portions and one or more cyclic portions, which may be selected from carbocyclic, aryl and heterocyclyl groups. The hydrocarbyl group includes monovalent groups and polyvalent groups as specified and may, for example, include one or more groups selected from alkyl, alkenyl, alkynyl, carbocyclyl (e.g. cycloalkyl or cycloalkenyl), aryl and heterocyclyl. The hydrocarbyl group may contain one or more heteroatoms, such as oxygen, nitrogen, sulphur, silicon or halogen which may be part of a functional group such as an alcohol, ether, carbonyl, ester, carboxylic acid, carbonate, amide, amine, carbamate, urea, thiol, thioether, thioester, thioacid, thioamide, silane organic halide or heterocycle, the hydrocarbyl linker may contain any combination of the above insofar as it is chemically stable. Furthermore, in some embodiments, halogens may entirely replace the hydrogen component of the hydrocarbyl group (i.e. the carbon-bonded hydrogens) to give the corresponding halo-substituted analogue.

The term "alkyl" as used herein refers to a monovalent straight- or branched-chain alkyl moiety. Unless specifically indicated otherwise, the term "alkyl" does not include optional substituents. The term "haloalkyl" as used herein refers to an alkyl group substituted with one or more halogen atoms. The term "halogen" as used herein refers to any of fluorine, chlorine, bromine, or iodine.

The term "alkyloxy" as used herein refers to an alkyl group substituted with one or more hydroxy groups or ether groups. The term "alkylamino" as used herein refers to an alkyl group substituted with one or more primary, secondary, or tertiary amine groups.

The term "cycloalkyl" as used herein refers to a monovalent saturated aliphatic hydrocarbyl moiety containing at least one ring, wherein said ring has at least 3 ring carbon atoms. The cycloalkyl groups mentioned herein may optionally have alkyl groups attached thereto. Examples of cycloalkyl groups include groups that are monocyclic, polycyclic (e.g., bicyclic) or bridged ring system. Examples of cycloalkyl groups include cyclopropyl, cyclobutyl, cyclopentyl, cyclohexyl and the like. The term "heterocycloalkyl" as used herein refers to a cycloalkyl group wherein the ring contains at least one heteroatom selected from oxygen, nitrogen, and sulphur. Examples of heterocycloalkyl groups include morpholine, piperidine, piperazine and the like.

The term "alkenyl" as used herein refers to a monovalent straight- or branched-chain alkyl group containing at least one carbon-carbon double bond, of either E or Z configuration unless specified. The term "alkynyl" as used herein refers to a monovalent straight- or branched-chain alkyl group containing at least one carbon-carbon triple bond. Examples of alkenyl groups include ethenyl, 2-propenyl, 1-butenyl, 2-butenyl, 3-butenyl, 1-pentenyl, 2-pentenyl, 3-pentenyl, 1-hexenyl, 2-hexenyl, 3-hexenyl and the like.

The term "aryl" as used herein refers to an aromatic carbocyclic ring system. An example of an aryl group includes a group that is a monocyclic aromatic ring system or a polycyclic ring system containing two or more rings, at least one of which is aromatic. Examples of aryl groups include aryl groups that comprise from 1 to 6 exocyclic carbon atoms in addition to ring carbon atoms. Examples of aryl groups include aryl groups that are monovalent or polyvalent as appropriate. Examples of monovalent aryl groups include phenyl, benzyl, naphthyl, fluorenyl, azulenyl, indenyl, anthryl and the like. An example of a divalent aryl group is 1,4-phenylene.

The term "heteroaryl" as used herein refers to an aromatic heterocyclic ring system wherein said ring atoms include at least one ring carbon atom and at least one ring heteroatom selected from nitrogen, oxygen and sulphur. Examples of heteroaryl groups include heteroaryl groups that are a monocyclic ring system or a polycyclic (e.g. bicyclic) ring system, containing two or more rings, at least one of which is aromatic. Examples of heteroaryl groups include those that, in addition to ring carbon atoms, comprise from 1 to 6 exocyclic carbon atoms. Examples of heteroaryl groups include those that are monovalent or polyvalent as appropriate. Examples of heteroaryl groups include pyridyl, pyrimidyl, thiopheneyl, isoxazolyl and benzo[b]furanyl groups.

The term "polyamine" as used herein refers to an organic compound having a plurality of amine groups. Preferably the polyamine is an organic compound having a plurality of amine groups having active hydrogens, i.e. one or more primary amines and/or secondary amines, also described herein as "nucleophilic amine(s)". For the avoidance of doubt, in the context of the present invention a 'diamine' having two amino groups is considered to fall within the scope of a "polyamine", and a 'monoamine' is having one amino group is not considered to fall within the scope of a "polyamine".

The term "copolymer" as described herein is used to describe a type of polymer, wherein the polymer is derived from more than one species of monomer.

The present invention relates to a hitherto unknown class of amido Mannich base compounds, examples of which include salicylamide Mannich bases. The compounds of the present invention are useful as hardeners in, for example, in epoxy resin systems.

The general formula of an amide Mannich base of the present invention is an aromatic or heteroaromatic core substituted with one or more Mannich amine groups, and with one or more amino substituted amides. The compounds of the present invention may be monomeric or polymeric with a repeating unit as described herein within a polymer or co-polymer of said repeating units. The aromatic or heteroaromatic core may also be substituted with additional substituents as defined hereinbelow.

In the context of the present invention the "Mannich amine group" is a secondary or tertiary amine group bonded to the aromatic or heteroaromatic core via an unsubstituted or substituted methylene linker (e.g. -CH₂- or -CR₂-). Said amine group is substituted with at least one hydrocarbyl group as defined hereinbelow, preferably wherein at least one of said hydrocarbyl groups is substituted with at least one amine, more preferably a primary or secondary amine.

In the context of the present invention the "amino substituted amide" refers to an amide group bonded at its carbonyl carbon directly to the aromatic or heteroaromatic core. The amide nitrogen is substituted with at least one hydrocarbyl group as defined hereinbelow, wherein at least one of said hydrocarbyl groups is substituted with at least one primary or secondary amine.

The amide Mannich base of the present invention thus comprises at least one nucleophilic amine group. A nucleophilic amine group is a primary or secondary amine group which is capable of performing nucleophilic attack to react with an electrophile, for example the epoxide group of an epoxy resin. As the at least one amino substituted amide comprises at least one nucleophilic amine, the amide Mannich base of the present invention may react with epoxy resin to act as a hardener/curative, for example, by causing crosslinking. The potential for reaction with epoxy resins may be increased by the presence of further nucleophiles which may be present in the compounds of the present invention. For example, further primary or secondary amines may be present on the at least one amino substituted amide, and/or Mannich amine group, as well as phenolic hydroxy group(s) on the aromatic or heteroaromatic core.

The compounds of the present invention all comprise an aromatic or heteroaromatic core derived from a carboxylic acid, or ester thereof. This provides the additional advantage of being able to utilise renewable bio-based feedstocks. Certain naturally occurring aromatic or heteroaromatic carboxylic acids may provide the additional advantage of increasing the bio-based content of the compound formed therefrom. For example, salicylic acid is derived from willow bark, and various anacardic acids may be derived from cashew nut shell liquid, which are 2-hydroxy benzoic acids substituted with C₁₅ to C₁₇ alkyl or alkenyl chain. Similarly, 2-furoic acid may be derived from furfural, biocatalytically.

Another advantage provided by the compounds of the present invention is the replacement of phenol as a starting material. As would be appreciated, phenol is a toxic and polluting material. Additionally, residual phenol may be leftover in the end product when phenol is used as a starting material, thus contributing to toxicity of a phenol derived epoxy resin hardener. The present compounds may be prepared using innocuous, as well as naturally occurring feedstocks such as salicylic acid and 2-furoic acid in place of phenol. Thus, the compounds of the present invention offer a significantly improved hazard classification over analogous phenol-based curatives (and avoids the toxicity issues associated with epoxy resins obtained using phenol-based curatives), whilst maintaining the useful properties associated with a conventional Mannich base curing agent.

In one aspect, the present invention provides a compound of formula (2): 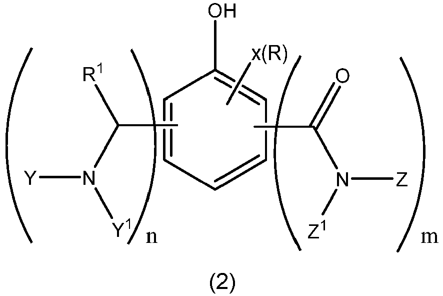 wherein:
each R group is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkyloxy, C₁ to C₂₀ alkylamino, -OH, -OR², -NH₂, -NHR², - NR²₂, -C(O)OH, -C(O)OR², -C(O)NH₂, -C(O)NHR², -C(O)NR²₂, -O(CO)H, -O(CO)R², -NH(CO)H, -NH(CO)R², -NR²(CO)H, -NR²(CO)R², -SH, -SR², -SO₂H, -SO₂R², - SO₃R², -SO₃H, -SiR²₃, -NO₂, -CN, -F, -Cl, -Br, and -I;
each R¹ group is independently selected from: -H, C₁ to C₁₀ alkyl, C₄ to C₁₀ haloalkyl, C₃ to C₁₂ cycloalkyl, C₂ to C₁₀ alkenyl, C₂ to C₁₀ alkynyl, C₆ to C₁₂ aryl, and C₃ to C₁₂ heteroaryl;
each R² is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkoxy, and C₁ to C₂₀ alkylamino;
Y is independently selected from a C₁ to C₂₅₀ hydrocarbyl group;
Y¹ is independently selected from hydrogen or a C₁ to C₂₅₀ hydrocarbyl group;
Z is independently selected from a C₂ to C₂₅₀ hydrocarbyl group comprising at least one primary or secondary amine;
Z¹ is independently selected from hydrogen or a C₁ to C₂₅₀ hydrocarbyl group;
n is independently 1, 2 or 3;
m is independently 1 or 2;
x is independently 0 to 3;
wherein n + m + x = 2 to 5; and
wherein the term hydrocarbyl refers to a monovalent group comprising a major proportion (i.e., more than 50 %) of hydrogen and carbon atoms, preferably consisting exclusively of hydrogen and carbon atoms.

In preferred embodiments, the total combined number of Mannich amine groups (represented by (6) below) and amino substituted amides (represented by (7) below) is 3, and preferably wherein the Mannich amine groups and amino substituted amides are substituted on the benzene ring in the 2,4,6-positions relative to the hydroxyl group. In such embodiments, the compound has the formula (2a): 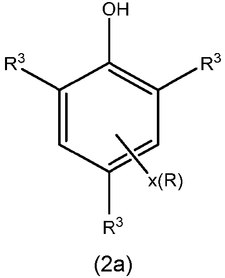 wherein:
each R³ is independently either an amine substituent of formula (6) or an amide substituent of formula (7), and at least one R³ is the amine substituent of formula (6) and at least one R³ is the amide substituent of formula (7): 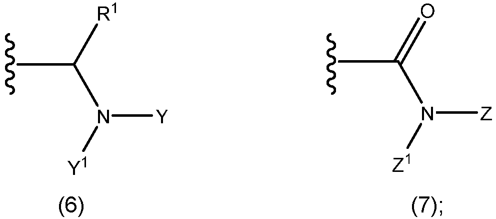
R, R¹, Y, Y¹, Z, and Z¹ are each as defined for the compound of formula (2); and
x is independently 0 to 2.

In preferred embodiments, the total combined number of Mannich amine groups (represented by (6) below) and amino substituted amides (represented by (7) below) is 4, and preferably wherein the Mannich amine groups and amino substituted amides are substituted on the benzene ring in the 2,4,6-positions relative to the hydroxyl group, with an amino substituted amide at the 3-position relative to the hydroxyl group. In such embodiments, the compound has the formula (2b): 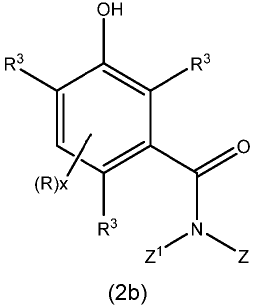 wherein:
each R³ is independently either an amine substituent of formula (6) or an amide substituent of formula (7), and at least one R³ is the amine substituent of formula (6); 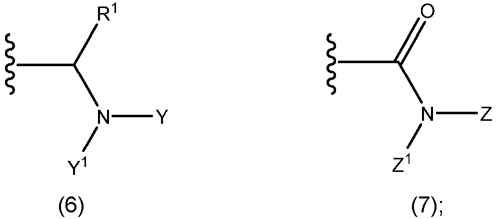
R, R¹, Y, Y¹, Z, and Z¹ are each as defined for the compound of formula (2); and
x is independently 0 or 1.

In preferred embodiments, the benzene ring is substituted with two Mannich amine groups and one amino substituted amide substituted on the benzene ring in the 2,4,6-positions relative to the hydroxyl group. In some embodiments, the one amino substituted amide is at the 2-position, in other embodiments the one amino substituted amide is at the 4-position. In such embodiments, the compound has the formula (2a-i) or (2a-ii): 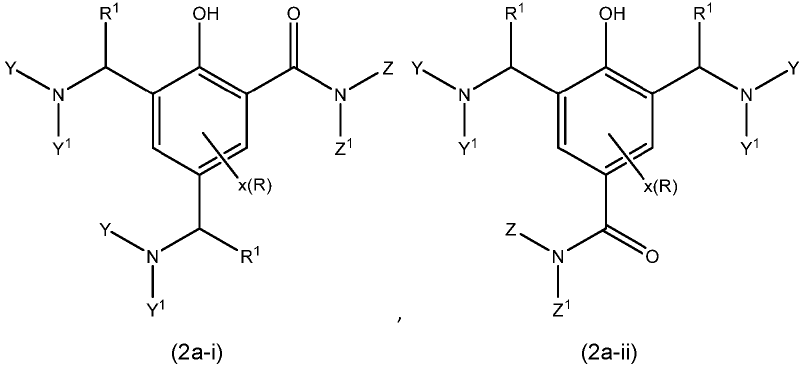 wherein:
R, R¹, Y, Y¹, Z and Z¹ are each as defined for the compound of formula (2); and
x is independently 0 to 2.

### Polymeric Compounds

The compound of the present invention may be a novolak type polymer or copolymer comprising a repeating unit of formula (4) defined hereinbelow. A novolak type polymer or copolymer is a polymer prepared or preparable by the condensation reaction of a ketone or aldehyde with a phenolic compound, which in the context of the present invention is an amide Mannich base compound having a benzene ring core substituted with at least one hydroxyl group. The resulting polymer thus comprises repeating units of an amide Mannich base having a benzene ring core substituted with at least one hydroxyl group, linked by a methylene group (defined hereinbelow as L) which is bonded to the benzene ring and to the benzene ring of the adjacent repeating unit. The methylene linker may be unsubstituted, i.e. be bonded to two hydrogen atoms (-CH₂-), or substituted with one or two substituents as defined as R⁹ and R¹⁰ (-CR⁹R¹⁰-), hereinbelow. As would be appreciated, the substituents on the methylene linker are determined by the choice of ketone or aldehyde used in the polycondensation reaction. During the polycondensation reaction the ketone or aldehyde preferentially react at benzene ring positions which are favoured due to steric and mesomeric effects from the other substituents on the benzene ring, thus, the polymer or copolymer comprising a repeating unit of formula (4) defined hereinbelow is not necessarily limited to any specific substitution pattern. As a consequence, in a polymer or copolymer comprising a repeating unit of formula (4) defined hereinbelow, the substitution pattern of the methylene linker on the benzene ring is not necessarily consistent across subsequent repeating units.

The polymer or copolymer comprising a repeating unit of formula (4) defined hereinbelow may be a straight chain or may comprise some degree of branching. Formula (4) defines a repeating unit having one methylene linker (L) and one trailing bond to the methylene linker (L) of the adjacent repeating unit, thus forming part of a straight chain or linear polymer. The polymer or copolymer comprising a repeating unit of formula (4) defined hereinbelow may also be a branched polymer comprising one or more repeating units having two single carbon liners or two trailing bonds to single carbon linkers of adjacent repeating units.

The polymer or copolymer comprising a repeating unit of formula (4) defined hereinbelow further comprises at least two end-groups at the polymer terminals.

In one aspect, the present invention provides a polymeric or co-polymeric compound comprising a repeating unit of formula (4): 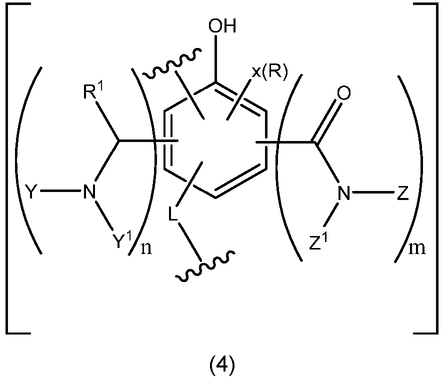 wherein:
R is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkyloxy, C₁ to C₂₀ alkylamino, -OH, -OR², -NH₂, -NHR², -NR²₂, -C(O)OH, -C(O)OR², -C(O)NH₂, -C(O)NHR², -C(O)NR²₂, -O(CO)H, -O(CO)R², -NH(CO)H, -NH(CO)R², -NR²(CO)H, -NR²(CO)R², -SH, -SR², -SO₂H, -SO₂R², -S0_{3R}², -SO₃H, -SiR²₃, -NO₂, -CN, -F, -Cl, -Br, and -I;
each R¹ group is independently selected from: -H, C₁ to C₁₀ alkyl, C₁ to C₁₀ haloalkyl, C₃ to C₁₂ cycloalkyl, C₂ to C₁₀ alkenyl, C₂ to C₁₀ alkynyl, C₆ to C₁₂ aryl, and C₃ to C₁₂ heteroaryl;
each R² is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkoxy, and C₁ to C₂₀ alkylamino;
Y is independently selected from a C₁ to C₂₅₀ hydrocarbyl group;
Y¹ is independently selected from hydrogen or a C₁ to C₂₅₀ hydrocarbyl group;
Z is independently selected from a C₂ to C₂₅₀ hydrocarbyl group comprising at least one primary or secondary amine;
Z¹ is independently either hydrogen or a C₄ to C₂₅₀ hydrocarbyl group; and
L is a linking group which connects the repeating unit to a single adjacent repeating unit of the polymer or co-polymer, and is selected from -CR⁹R¹⁰-;
each represents the attachment point between the linking group, L, of one repeating unit and a single adjacent repeating unit;
each R⁹ group is independently selected from: -H, C₁ to C₁₀ alkyl, C₁ to C₁₀ haloalkyl, C₃ to C₁₂ cycloalkyl, C₂ to C₁₀ alkenyl, C₂ to C₁₀ alkynyl, C₆ to C₁₂ aryl, C₃ to C₁₂ heteroaryl, and -C(O)H;
each R¹⁰ group is independently selected from: -H, C₁ to C₁₀ alkyl, C₁ to C₁₀ haloalkyl, C₃ to C₁₂ cycloalkyl, C₂ to C₁₀ alkenyl, C₂ to C₁₀ alkynyl, C₆ to C₁₂ aryl, and C₃ to C₁₂ heteroaryl;
or R⁹ and R¹⁰ are taken together to form a C₃ to C₁₂ cycloalkyl;
n is independently 1 or 2;
m is independently 1 or 2;
x is independently 0 or 1;
with the proviso that n + m + x = 2 or 3; and
wherein the term hydrocarbyl refers to a monovalent group comprising a major proportion (i.e., more than 50 %) of hydrogen and carbon atoms, preferably consisting exclusively of hydrogen and carbon atoms.

In preferred embodiments, the polymeric or co-polymeric compound comprises a repeating unit wherein the amino substituted amide is at the 2-position and/or the 4-position relative to the hydroxyl group. In such embodiments, the polymeric or co-polymeric compound comprises a repeating unit of formula (4a) and/or (4b): 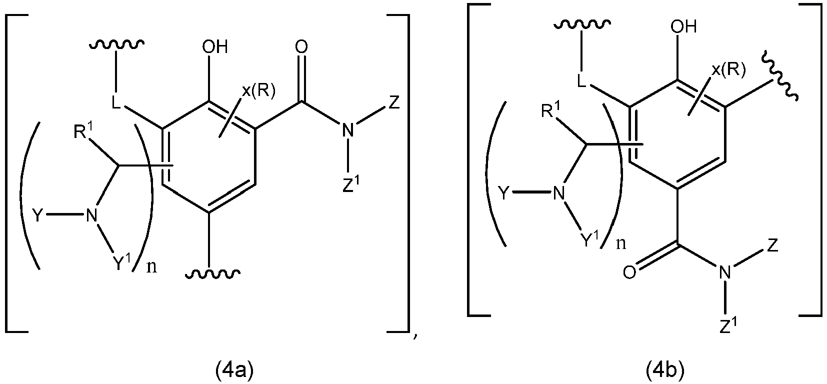 wherein:
L, 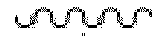 , R, R¹, R², Y, Y¹, Z, and Z¹ are as defined for the compound of formula (4) above;
n is independently 1 or 2;
x is independently 0 or 1; and
with the proviso that n + x = 1 or 2.

When the ring positions of other substituents are fixed, the ring position at which the methylene linker L is formed by polycondensation reaction may be more predictable. The hydroxyl group is *ortho, para* directing, and the amino substituted amide (as well as the carboxylic acid from which it may be prepared) is *meta* directing. This means that the polymeric or co-polymeric compound comprising a repeating unit of formula (4a) and/or (4b) may be reliably prepared from the appropriate starting materials. For example, suitable starting materials for repeating units (4a) and (4b) are 2-hydroxybenzoic acid and 4-hydroxybenzoic acid, respectively. In each of these starting materials, the polycondensation reaction will occur predominantly at the available meta and para positions relative to the hydroxyl group, thus providing the substitution pattern defined in (4a) and (4b), respectively. One or two Mannich amine groups may then be prepared at one or both of the available ring positions by use of the Mannich reaction.

Where 3-hydroxybenzoic acid is used as a starting material a more complex set of isomers may be prepared as the hydroxyl and carboxylic acid group direct to competing positions. As there are four available positions for the polycondensation reaction to occur there are six possible isomers with regard to the position of the single carbon linker L and the attachment point between the linking group L, of one repeating unit and a single adjacent repeating unit. The substitution pattern for these groups may be any one of 2, 4-; 2, 5-; 2, 6-; 4, 5-; 4, 6-; and 5, 6-positions relative to the hydroxyl group). One or two Mannich amine groups may then be prepared at one or both of the available ring positions by use of the Mannich reaction.

In some embodiments, the polymeric or co-polymeric compound comprises a repeating unit wherein the amino substituted amide is at the 3-position relative to the hydroxyl group. In such embodiments, the polymeric or co-polymeric compound comprises a repeating unit according to formula (4) wherein the amino substituted amide is at the 3-position relative to the hydroxyl group; the single carbon linker L and the attachment point between the linking group L, of one repeating unit and a single adjacent repeating unit, are at any one of the 2, 4-; 2, 5-; 2, 6-; 4, 5-; 4, 6-; or 5, 6-positions relative to the hydroxyl group.

In some embodiments, an R group may be present. Typically, where an R group is present, it will exist on the hydroxyl benzoic acid, or ester thereof, starting material prior to polycondensation. The directing nature and the position of the R group would also impact the position at which the polycondensation reaction takes place, as would be appreciated by the skilled person.

In preferred embodiments of any of the novolak type polymer or co-polymer compounds, the methylene linker L is CR⁹R¹⁰;
wherein:
each R⁹ group is independently selected from: -H, C₁ to C₁₀ alkyl, C₃ to C₁₂ cycloalkyl, C₂ to C₁₀ alkenyl, C₆ to C₁₂ aryl and C₃ to C₁₂ heteroaryl, preferably wherein each R⁹ group is independently: -H or furan I; and
each R¹⁰ group is -H.

In this embodiment, an aldehyde is used in polycondensation reaction, thus limiting R¹⁰ to hydrogen.

A polymeric or co-polymeric compound comprising a repeating unit of formula (4), (4a) or (4b) typically has a degree of polymerisation (P), i.e. the number of monomeric units in the polymeric or co-polymeric compound of from 3 to 40. In some embodiments, P is from, 4 to 20, preferably from 5 to 15. Adjusting P as desired would be within the capabilities of the skilled person. As would be appreciated, P may be adjusted to fine tune properties such as the melting point or viscosity of the polymeric or co-polymeric compound.

A co-polymeric compound comprising a repeating unit of formula (4) may comprise, consist, or consist essentially of multiple different repeating units within the scope of formula (4), (4b) and/or (4b). In some embodiments, the co-polymeric compound comprising a repeating unit of formula (4) may further comprise repeating units not within the scope of formula (4) which are derived from polycondensation rection of phenolic compounds and aldehydes or ketones.

In one embodiment, the compound is a polymer consisting of, or consisting essentially of, the repeating units of formula (4), and end-groups.

The compound of the present invention may be a vinyl polymer comprising a repeating unit of formula (5) defined hereinbelow. In the context of the present invention a vinyl polymer or copolymer is a polymer prepared or preparable by the polymerisation a carbon-carbon double bonds. In the context of the present invention, the relevant monomer is an amide Mannich base having a benzene ring core substituted with at least one hydroxyl group and with at least one substituent comprising a carbon-carbon double bond.

The resulting polymer thus comprises repeating units of an amide Mannich base a having a benzene ring core substituted with at least one hydroxyl group, linked to an alkane chain backbone. The alkane chain backbone may be unsubstituted, i.e. be bonded to three hydrogen atoms at positions R⁴, R⁵ and R⁶, or substituted with one to three substituents as defined hereinbelow. Similarly, the benzene ring may be directly bonded to the alkane chain backbone, or bonded by another group, as defined hereinbelow. As would be appreciated, the substituents of the alkane chain backbone are determined by substituents present on the vinyl group prior to polymerisation.

The polymer or copolymer comprising a repeating unit of formula (5) defined hereinbelow further comprises two end-groups at the polymer terminals.

In one aspect, the present invention provides a polymeric or co-polymeric compound comprising a repeating unit of formula (5): 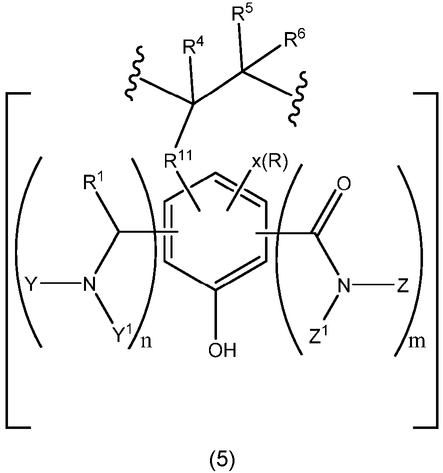 wherein:
R is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkyloxy, C₁ to C₂₀ alkylamino, -OH, -OR², -NH₂, -NHR², -NR²₂, - C(O)OH, -C(O)OR², -C(O)NH₂, -C(O)NHR², -C(O)NR²₂, -O(CO)H, -O(CO)R², - NH(CO)H, -NH(CO)R², -NR²(CO)H, -NR²(CO)R², -SH, -SR², -SO₂H, -SO₂R², - SO₃R², -SO₃H, -SiR²₃, -NO₂, -CN, -F, -Cl, -Br, and -I;
each R¹ group is independently selected from: -H, C₁ to C₁₀ alkyl, C₁ to C₁₀ haloalkyl, C₃ to C₁₂ cycloalkyl, C₂ to C₁₀ alkenyl, C₂ to C₁₀ alkynyl, C₆ to C₁₂ aryl, and C₃ to C₁₂ heteroaryl;
each R² is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkoxy, and C₁ to C₂₀ alkylamino;
Y is independently a C₁ to C₂₅₀ hydrocarbyl group;
Y¹ is independently either hydrogen or a C₁ to C₂₅₀ hydrocarbyl group;
Z is independently a C₂ to C₂₅₀ hydrocarbyl group comprising at least one primary or secondary amine;
Z¹ is independently either hydrogen or a C₁ to C₂₅₀ hydrocarbyl group; and R⁴, R⁵ and R⁶ are independently selected from: -H, C₁ to C₁₀ alkyl, -F, -Cl, -Br, -I, -OH, -OR¹, -CN, -C(O)OH, -C(O)OR¹, -C(O)NH₂, -C(O)NHR¹, -C(O)NR¹₂, -O(CO)H, or -O(CO)R¹;
each 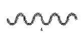 represents an attachment point between one repeating unit and a single adjacent repeating unit;
R¹¹ is either a direct bond or -OC(O)-;
n is independently 1, 2 or 3;
m is independently 1, 2 or 3;
x is independently 0 to 2;
with the proviso that n + m + x = 2 to 4; and
wherein the term hydrocarbyl refers to a monovalent group comprising a major proportion (i.e., more than 50 %) of hydrogen and carbon atoms, preferably consisting exclusively of hydrogen and carbon atoms.

In a preferred embodiment, the polymeric or co-polymeric compound comprises a repeating unit of formula (5a): 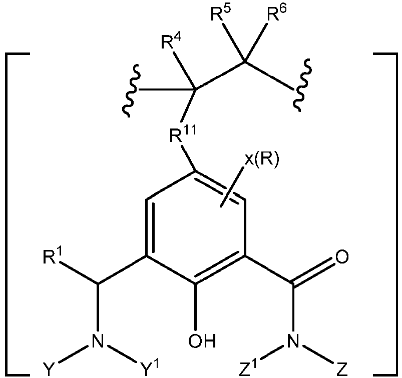 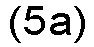 wherein:
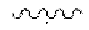 , R, R¹, R⁴, R⁵, R⁶, Y, Y¹, Z, Z¹ and R¹¹ are as defined for the compound of formula (5); and
x is independently 0, 1 or 2.

In the case of formula (5a), the starting material is for example salicylic acid substituted at the 4-position relative to the hydroxyl group, with -R¹¹-CR⁴=CR⁵R⁶, or a preformed vinyl polymer thereof. Amidation and the Mannich reaction may then be performed as described above.

As would be appreciated, other substitution patterns within the scope of formula (5) may be prepared from the appropriate starting materials.

The nature of the substituents, if any, at R⁴, R⁵ and R⁶ are dependent on the substituents, if any, present on the vinyl monomer starting material. In a preferred embodiment, R⁴, R⁵ and R⁶ are independently selected from: -H, C₁ to C₁₀ alkyl, -F, -Cl, - Br, -I, and -CN; more preferably wherein R⁴ is -Me, and R⁵ and R⁶ are -H, more preferably wherein R⁴, R⁵ and R⁶ and -H.

The nature of the R¹¹ group depends on if the monomeric vinyl starting material has a vinyl group directly bonded to the benzene ring, or a vinyl group bonded to the benzene ring via an -OC(O)- group, i.e. where the benzene ring bears an acrylate group.

Vinyl type polymers typically have larger degrees of polymerisation (P) than novolak type polymers. A polymeric or co-polymeric compound comprising a repeating unit of formula (5) or (5a) typically has a P of from 3 to 1000. In some embodiments, P is from, 3 to 200, preferably from 3 to 100, more preferably from 3 to 40, even more preferably from 4 to 20, even more preferably from 5 to 15. Adjusting P as desired would be within the capabilities of the skilled person. As would be appreciated, P may be adjusted to fine tune properties such as the melting point or viscosity of the polymeric or co-polymeric compound.

A co-polymeric compound comprising a repeating unit of formula (5) may comprise, consist, or consist essentially of multiple different repeating units within the scope of formula (5) and/or (5a). In some embodiments, the co-polymeric compound comprising a repeating unit of formula (5) may further comprise repeating units not within the scope of formula (5) which are derived from alkenes. For example, the co-polymer may further comprise any one or more of the following repeating units [-CH₂-CH₂-], [-CH₂-CH(CH₃)-], [-[CF₂-CF₂-], [-CH₂-CHCl-], [-CH₂-CH(O(CO)CH₃)-], [-CH₂-CHOH-], [-CH₂-CHPh-] and [-CH₂-CHCN-].

In one embodiment, the compound is a polymer consisting of, or consisting essentially of, repeating units of formula (5), and end-groups.

### General substituents

In all of the compounds of the present invention, including all compounds within the scope of formula (2), as well as all polymeric or co-polymeric compounds comprising repeating units of formula (4) or formula (5), at least one Mannich amine group, and at least one amino substituted amide group is present.

The amino group of the Mannich amine group is either a secondary or tertiary amine. The amino group is bonded to the single carbon atom connected to the aromatic or heteroaromatic core; a hydrocarbyl group defined hereinabove as Y; and a group defined hereinabove as Y¹. Y¹ may either be a hydrocarbyl group as defined hereinabove, or hydrogen. In the case that the amino group of the Mannich amine group is secondary amine, Y¹ is hydrogen. In the case that the amino group of the Mannich amine group is tertiary amine, Y¹ is a hydrocarbyl group.

Each Y is independently selected from a C₁ to C₂₅₀ hydrocarbyl group, preferably comprising at least one amine, more preferably at least one primary or secondary amine. Each Y¹ is independently selected from hydrogen or a C₁ to C₂₅₀ hydrocarbyl group.

In preferred embodiments, each Y is independently selected from a C₂ to C₁₅₀ hydrocarbyl group (e.g. a C₃ to C₇₅ or C₅ to C₄₀ hydrocarbyl group), preferably comprising at least one amine, more preferably at least one primary or secondary amine.

In some embodiments, each Y¹ is independently selected from a C₃ to C₁₅₀ hydrocarbyl group (e.g. a C₃ to C₇₅ or C₅ to C₄₀ hydrocarbyl group) preferably comprising at least one amine, more preferably at least one primary or secondary amine. In alternative embodiments Y¹ is hydrogen.

In some embodiments, each Y group is the same. In some embodiments, each Y¹ group is the same. In some embodiments, each Y group is the same and each Y¹ group is the same.

As would be appreciated, the Mannich amine group comprises a methylene group which is derived from the aldehyde used in the Mannich reaction from which the Mannich amine group is synthesised. The substituent on the methylene group, defined hereinabove as R¹, is derived from the substituent present the aldehyde used in the Mannich reaction from which the Mannich amine group is synthesised. In the case that formaldehyde is used in the Mannich reaction, R¹ will be hydrogen.

Each R¹ group is independently selected from: -H, C₁ to C₁₀ alkyl, C₁ to C₁₀ haloalkyl, C₃ to C₁₂ cycloalkyl, C₂ to C₁₀ alkenyl, C₂ to C₁₀ alkynyl, C₆ to C₁₂ aryl, and C₃ to C₁₂ heteroaryl.

In the case that furfural is used in the Mannich reaction, R¹ will be furan. In a preferred embodiment, the R¹ group is furan. Furfural is an inexpensive, renewable and naturally occurring feedstock derived from the dehydration of sugars, and occurs in a variety of agricultural by-products, including corncobs, oats, wheat bran, and sawdust. The use of furfural in preparation of compounds of the present invention therefore provides the advantage of introducing a degree of bio-based carbon content and utilising renewable feedstocks. Furfural is also advantageous in the replacement of formaldehyde which is highly toxic and environmentally polluting.

Each R¹ group is selected independently and may be either the same or different in the case of multiple Mannich amine groups. In preferred embodiments, all R¹ groups are the same. Different R¹ groups may be provided by subsequent Mannich reactions using different aldehydes.

In some embodiments, each R¹ group is the same.

In some embodiments, each Mannich amine is the same, i.e. each set of Y, Y¹ and R¹ are the same.

The amide group of amino substituted amide is either a secondary or tertiary amide. The amide nitrogen is bonded to the amide carbonyl carbon; a hydrocarbyl group defined hereinabove as Z; and a group defined hereinabove as Z¹. Z¹ may either be a hydrocarbyl group as defined hereinabove, or hydrogen. In the case that the amide group of the amino substituted amide is secondary amide, Z¹ is hydrogen. In the case that the amide group of the amino substituted amide is tertiary amide, Z¹ is a hydrocarbyl group.

Each Z is independently selected from a C₂ to C₂₅₀ hydrocarbyl group comprising at least one primary or secondary amine. Z¹ is independently selected from hydrogen or a C₁ to C₂₅₀ hydrocarbyl group.

In preferred embodiments, each Z is independently selected from a C₃ to C₁₅₀ hydrocarbyl group comprising at least one primary or secondary amine, more preferably from C₃ to C₇₅, even more preferably from C₅ to C₄₀.

In some embodiments, each Z¹ is independently selected from a C₃ to C₁₅₀ hydrocarbyl group comprising at least one primary or secondary amine, more preferably from C₃ to C₇₅, even more preferably from C₅ to C₄₀. In alternative embodiments Z¹ is hydrogen.

In some embodiments, each Z group is the same. In some embodiments, each Z¹ group is the same. In some embodiments, each amino substituted amide is the same, i.e. each Z group is the same and each Z¹ group is the same.

In some embodiments, Z and Y are the same. In some embodiments, Z¹ and Y¹ are the same. In some embodiments, Z and Y are the same and Z¹ and Y¹ are the same.

In preferred embodiments each Y and/or each Z are independently selected from:
a hydrocarbyl chain having the formula: -(R⁷-NH-)_{q}-H, wherein each -(R⁷-NH-)_{q}-H group independently satisfies one of the following conditions i) to iii):
i) R⁷ = a C₂ to C₁₂ alkyl group, and q = 1 to 7;
ii) R⁷ = a C₃ to C₁₈ cycloalkyl group, and q = 1 to 3; or
iii) R⁷ = a C₆ to C₁₈ aromatic or alkylaromatic group, and q = 1 to 3.

In preferred embodiments each Y and/or each Z are independently selected from a group of formula -(R⁷-NH-)_{q}-(CO)-R⁸-(CO)NH-(R⁷-NH-)_{q}-H;
wherein:
each R⁸ is independently a C₆ to C₇₀ branched or straight chain alkyl, cycloalkyl, alkenyl, or cycloalkenyl group, preferably wherein each R⁸ is independently a C₃₀ to C₃₆ branched chain alkyl, cycloalkyl, alkenyl, or cycloalkenyl group; and
each -(R⁷-NH-)_{q} moiety independently satisfies one of the following conditions i) to iii):
   i) R⁷ = a C₂ to C₁₂ alkyl group; and q = 1 to 7;
   ii) R⁷ = a C₃ to C₁₈ cycloalkyl group, and q = 1 to 3; or
   iii) R⁷ = a C₆ to C₁₈ aromatic or alkylaromatic group, and q = 1 to 3.

In such embodiments, the Y or Z group in question may for example, be prepared by reaction of an amine having the formula H₂N-(R⁷-NH-)_{q}-H with a dimer acid having the formula HO₂C-R⁸-CO₂H, for example in an approximately 2:1 molar ratio. Said Y or Z group may also be prepared by reaction of said dimer acid with an existing Z or Y group already attached to a compound of the present invention and having the formula -(R⁷-NH-)_{q}-H, followed by subsequent reaction with another amine of the formula H₂N-(R⁷-NH-)_{q}-H.

In preferred embodiments each Y and/or each Z are independently selected from:
a hydrocarbyl chain having the formula: -(R⁷)_{q}-CH₂CH(CH₃)NH₂, wherein each -(R⁷)q-CH₂CH(CH₃)NH₂ group independently satisfies one of the following conditions:
i) R⁷ = a C₂ to C₃ ether group, and q = 1 to 80; or
ii) R⁷ = a C₄ to C₈ ether group, and q = 1 to 30.

In such embodiments, the Y or Z group in question is derived from a Jeffamine (RTM) which are commercially available.

In preferred embodiments each Y¹ and/or each Z¹ are independently selected from -H, - Me, -Et, -CH₂CH₂NH₂, -CH₂CH₂NHCH₂CH₂NH₂, and -(R⁷-NH-)_{q}-H, wherein R⁷ is a C₂ to C₁₂ alkyl group, and q = 1 to 3; preferably wherein each Y¹ and/or each Z¹ are -H.

In all of the compounds of the present invention, including all compounds within the scope of formula (2), as well as all polymeric or co-polymeric compounds comprising repeating units of formula (4) or formula (5), the aromatic or heteroaromatic core may optionally be substituted with one or more further substituents, defined herein as R, where the required ring positions are available for substitution.

Each R group is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkyloxy, C₁ to C₂₀ alkylamino, -OH, -OR², -NH₂, -NHR², -NR²₂, -C(O)OH, - C(O)OR², -C(O)NH₂, -C(O)NHR², -C(O)NR²₂, -O(CO)H, -O(CO)R², -NH(CO)H, - NH(CO)R², -NR²(CO)H, -NR²(CO)R², -SH, -SR², -SO₂H, -SO₂R², -S0_{3R}², -SO₃H, -SiR²₃, -NO₂, -CN, -F, -Cl, -Br, and -I; each R² is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkoxy, and C₁ to C₂₀ alkylamino.

In preferred embodiments, each R group is independently selected from: C₁ to C₂₀ alkyl, C₃ to C₂₀ cycloalkyl, C₂ to C₂₀ alkenyl, C₂ to C₂₀ alkynyl, C₁ to C₂₀ alkyloxy, C₁ to C₂₀ alkylamino, -OH, -OR², -NH₂, -NHR², -NR²₂, -C(O)NH₂, -C(O)NHR², -C(O)NR²₂, - O(CO)H, -O(CO)R⁴, -NH(CO)H, -NH(CO)R², -NR²(CO)H, -NR⁴(CO)R², -SH, -SR², - SiR²₃, -F, -Cl, -Br, and -I; each R² is independently selected from: C₁ to C₂₀ alkyl, C₃ to C₂₀ cycloalkyl, C₂ to C₂₀ alkenyl, C₂ to C₂₀ alkynyl, C₁ to C₂₀ alkoxy, and C₁ to C₂₀ alkylamino;
preferably wherein each R group is independently selected from: C₁ to C₁₀ alkyl, C₃ to C₁₀ cycloalkyl, C₂ to C₁₀ alkenyl, C₂ to C₁₀ alkynyl, C₁ to C₁₀ alkyloxy, C₁ to C₁₀ alkylamino, -OH, -OR², -NH₂, -NHR², and -NR²₂; and wherein each R² is independently selected from: C₁ to C₁₀ alkyl, C₃ to C₁₀ cycloalkyl, C₂ to C₁₀ alkenyl, C₂ to C₁₀ alkynyl, C₁ to C₁₀ alkoxy, and C₁ to C₁₀ alkylamino; more preferably wherein R is -OH.

In some embodiments of the compound of formula (2), the Z substituent may comprise an additional aromatic or heteroaromatic amide Mannich base so as to form a dimeric compound. In some such embodiments, the Z substituent is of formula (8), shown below. 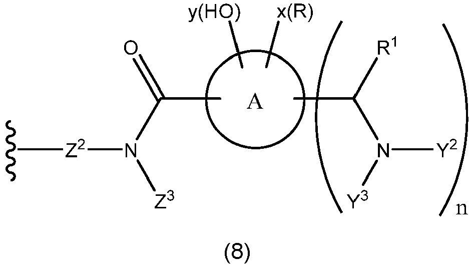
wherein Z² is a divalent C₂ to C₁₀₀ hydrocarbyl group;
wherein Z² is selected from hydrogen or a C₁ to C₅₀ hydrocarbyl group;
wherein Y² is independently selected from a C₂ to C₅₀ hydrocarbyl group comprising at least one primary or secondary amine;
wherein Y³ is independently selected from hydrogen or a C₁ to C₅₀ hydrocarbyl group;
wherein 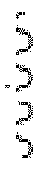 represents the attachment point between the Z substituent of formula (8) and the remainder of the compound of formula (2);
wherein n, y, x, R, and R¹ are as defined hereinabove in relation to the compound of formula (2), including any embodiments described hereinabove in relation thereto;
with the proviso that the total number of carbon atoms in the substituent of formula (8) is no more than 250;
and wherein ring A is selected from a benzene ring, a pyridine ring and a 5-membered heteroaromatic ring having one heteroatom.

In the substituent of formula (8), preferably, ring A is a benzene ring.

Compounds bearing a Z substituent of formula (8) have a high viscosity, however, it has been found that a small degree of dilution with a diluent, such as benzyl alcohol or xylene, can significantly reduce the viscosity of such compounds.

In a specific embodiment, one such compound bearing a Z substituent of formula (8), is a compound of formula (9) shown below. 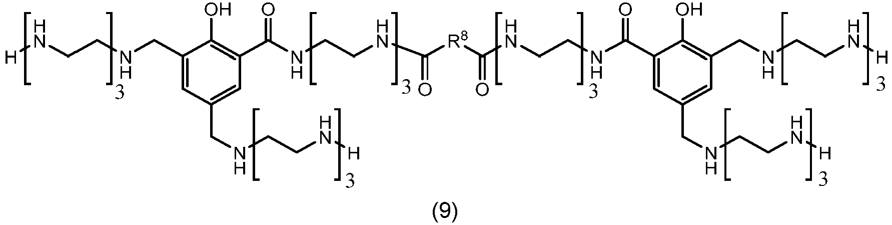 wherein R³ is a C₆ to C₇₀ branched or straight chain alkyl, cycloalkyl, alkenyl, or cycloalkenyl group, preferably wherein each R₈ is independently a C₃₀ to C₃₆ branched or straight chain alkyl, cycloalkyl, alkenyl, or cycloalkenyl group. For example, the R³ moiety may be derived from a dimer acid, such as a C₃₆ dimer acid.

In further embodiments, the present invention further relates to compounds, and methods of preparing said compounds, as follows:
1.1. A compound of formula (2), wherein each R group is independently selected from: C₁ to C₂₀ alkyl, C₃ to C₂₀ cycloalkyl, C₂ to C₂₀ alkenyl, C₂ to C₂₀ alkynyl, C₁ to C₂₀ alkyloxy, C₁ to C₂₀ alkylamino, -OH, -OR², -NH₂, -NHR², -NR²₂, -C(O)NH₂, - C(O)NHR², -C(O)NR²₂, -O(CO)H, -O(CO)R⁴, -NH(CO)H, -NH(CO)R², -NR²(CO)H, -NR⁴(CO)R², -SH, -SR², -SiR²₃, -F, -Cl, -Br, and -I; and each R² is independently selected from: C₁ to C₂₀ alkyl, C₃ to C₂₀ cycloalkyl, C₂ to C₂₀ alkenyl, C₂ to C₂₀ alkynyl, C₁ to C₂₀ alkoxy, and C₁ to C₂₀ alkylamino.
1.2. A compound of formula (2), wherein each R group is independently selected from: C₁ to C₁₀ alkyl, C₃ to C₁₀ cycloalkyl, C₂ to C₁₀ alkenyl, C₂ to C₁₀ alkynyl, C₁ to C₁₀ alkyloxy, C₁ to C₁₀ alkylamino, -OH, -OR², -NH₂, -NHR², and -NR²₂; and each R² is independently selected from: C₁ to C₁₀ alkyl, C₃ to C₁₀ cycloalkyl, C₂ to C₁₀ alkenyl, C₂ to C₁₀ alkynyl, C₁ to C₁₀ alkoxy, and C₁ to C₁₀ alkylamino.
1.3. A compound of formula (2), wherein each R group is independently selected from: C₁ to C₂₀ alkyl, C₂ to C₂₀ alkenyl, C₁ to C₂₀ alkyloxy, -OH, -OR², -NH₂, - NHR², -NR²₂, -F, -Cl, -Br, and -I; and each R² is independently selected from: C₁ to C₂₀ alkyl, C₂ to C₂₀ alkenyl, and C₁ to C₂₀ alkoxy.
1.4. A compound of formula (2), wherein each R group is independently selected from: C₁ to C₂₀ alkyl, C₂ to C₂₀ alkenyl, C₁ to C₂₀ alkyloxy, -OH, -OR², -F, -Cl, -Br, and -I; and each R² is independently selected from: C₁ to C₂₀ alkyl, C₂ to C₂₀ alkenyl, and C₁ to C₂₀ alkoxy.
1.5. A compound of formula (2), wherein each R group is independently selected from: C₁ to C₁₀ alkyl, C₂ to C₁₀ alkenyl, C₁ to C₁₀ alkyloxy, -OH, -OR², -F, -Cl, -Br, and -I; and each R² is independently selected from: C₁ to C₁₀ alkyl.
1.6. A compound of formula (2), wherein each R group is -OH.
1.7. A compound of any one of 1.1 to 1.6, where x = 0 to 3.
1.8. A compound of any one of 1.1 to 1.6, where x = 0 to 2.
1.9. A compound of any one of 1.1 to 1.6, where x = 0 to 1.
1.10. A compound of any one of 1.1 to 1.6, where x = 0.
1.11. A compound of any one of 1.1 to 1.8, wherein there is plurality of R groups (x is 2 or 3) and each R group is the same.
1.17. A compound of any one of 1.1 to 1.11, wherein m = 1.
1.18. A compound of any one of 1.1 to 1.11 and 1.17, wherein n = 1.
1.19. A compound of any one of 1.1 to 1.6 and 1.8 to 1.11, and 1.18, wherein m = 2.
1.20. A compound of any one of 1.1 to 1.6 and 1.8 to 1.11, and 1.17 wherein n = 2.
1.21. A compound of any one of 1.1 to 1.11 and 1.17 to 1.20, wherein Y' = H.
1.22. A compound of any one of 1.1 to 1.11 and 1.17 to 1.21, wherein Z¹ = H.
1.23. A compound of any one of 1.1 to 1.11 and 1.17 to 1.22, wherein each Y group is a C₂ to C₁₅₀ hydrocarbyl group.
1.24. A compound of any one of 1.1 to 1.11 and 1.17 to 1.22, wherein each Y group is a C₃ to C₇₅ hydrocarbyl group.
1.25. A compound of any one of 1.1 to 1.11 and 1.17 to 1.22, wherein each Y group is a C₄ to C₄₀ hydrocarbyl group.
1.26. A compound of any one of 1.1 to 1.11 and 1.17 to 1.22, wherein each Y group is a hydrocarbyl chain having the formula: -(R⁷-NH-)_{q}-H, wherein each -(R⁷-NH-)_{q}-H group independently satisfies one of the following conditions i) to iii):
   i) R⁷ = a C₂ to C₁₂ alkyl group, and q = 1 to 7;
   ii) R⁷ = a C₃ to C₁₈ cycloalkyl group, and q = 1 to 3; or
   iii) R⁷ = a C₆ to C₁₈ aromatic or alkylaromatic group, and q = 1 to 3.
1.27. A compound of any one of 1.1 to 1.11 and 1.17 to 1.22, wherein each Y group is a hydrocarbyl chain having the formula: -(R⁷)q-CH₂CH(CH₃)NH₂, wherein each - (R⁷)q-CH₂CH(CH₃)NH₂ group independently satisfies one of the following conditions:
   i) R⁷ = a C₂ to C₃ ether group, and q = 1 to 80; or
   ii) R⁷ = a C₄ to C₈ ether group, and q = 1 to 30
1.28. A compound of any one of 1.1 to 1.11 and 1.17 to 1.22, wherein each Y group is a group of formula -(R⁷-NH-)q-(CO)-R⁸-(CO)NH-(R⁷-NH-)q-H;
   wherein: each R⁸ is independently a C₆ to C₇₀ branched or straight chain alkyl, cycloalkyl, alkenyl, or cycloalkenyl group, preferably wherein each R⁸ is independently a C₃₀ to C₃₆ branched chain alkyl, cycloalkyl, alkenyl, or cycloalkenyl group; and each -(R⁷-NH-)q moiety independently satisfies one of the following conditions i) to iii):
   R⁷ = a C₂ to C₁₂ alkyl group; and q = 1 to 7;
   R⁷ = a C₃ to C₁₈ cycloalkyl group, and q = 1 to 3; or
   R ⁷ = a C₆ to C₁₈ aromatic or alkylaromatic group, and q = 1 to 3.
1.29. A compound of any one of 1.1 to 1.11 and 1.17 to 1.28, wherein each Z group is a C₃ to C₁₅₀ hydrocarbyl group comprising at least one primary or secondary amine.
1.30. A compound of any one of 1.1 to 1.11 and 1.17 to 1.28, wherein each Z group is a C₃ to C₇₅ hydrocarbyl group comprising at least one primary or secondary amine.
1.31. A compound of any one of 1.1 to 1.11 and 1.17 to 1.28, wherein each Z group is a C₃ to C₄₀ hydrocarbyl group comprising at least one primary or secondary amine.
1.32. A compound of any one of 1.1 to 1.11 and 1.17 to 1.28, wherein each Z group is a hydrocarbyl chain having the formula: -(R⁷-NH-)_{q}-H, wherein each -(R⁷-NH-)_{q}-H group independently satisfies one of the following conditions i) to iii):
   i) R⁷ = a C₂ to C₁₂ alkyl group, and q = 1 to 7;
   ii) R⁷ = a C₃ to C₁₈ cycloalkyl group, and q = 1 to 3; or
   iii) R⁷ = a C₆ to C₁₈ aromatic or alkylaromatic group, and q = 1 to 3.
1.33. A compound of any one of 1.1 to 1.11 and 1.17 to 1.28, wherein each Z group is a hydrocarbyl chain having the formula: -(R⁷)q-CH₂CH(CH₃)NH₂, wherein each - (R⁷)q-CH₂CH(CH₃)NH₂ group independently satisfies one of the following conditions:
   i) R⁷ = a C₂ to C₃ ether group, and q = 1 to 80; or
   ii) R⁷ = a C₄ to C₈ ether group, and q = 1 to 30
1.34. A compound of any one of 1.1 to 1.11 and 1.17 to 1.28, wherein each Z group is a group of formula -(R⁷-NH-)q-(CO)-R⁸-(CO)NH-(R⁷-NH-)q-H;
   wherein: each R⁸ is independently a C₆ to C₇₀ branched or straight chain alkyl, cycloalkyl, alkenyl, or cycloalkenyl group, preferably wherein each R⁸ is independently a C₃₀ to C₃₆ branched chain alkyl, cycloalkyl, alkenyl, or cycloalkenyl group; and each -(R⁷-NH-)q moiety independently satisfies one of the following conditions i) to iii):
   R⁷ = a C₂ to C₁₂ alkyl group; and q = 1 to 7;
   R⁷ = a C₃ to C₁₈ cycloalkyl group, and q = 1 to 3; or
   R⁷ = a C₆ to C₁₈ aromatic or alkylaromatic group, and q = 1 to 3.
1.35. A compound of any one of 1.1 to 1.11 and 1.17 to 1.34, wherein R¹ is selected from -H, C₁ to C₁₀ alkyl, C₁ to C₁₀ haloalkyl, C₃ to C₁₂ cycloalkyl, C₂ to C₁₀ alkenyl, C₂ to C₁₀ alkynyl, C₆ to C₁₂ aryl, and C₃ to C₁₂ heteroaryl.
1.36. A compound of any one of 1.1 to 1.11 and 1.17 to 1.34, wherein R¹ is selected from -H, C₁ to C₁₀ alkyl, C₆ to C₁₂ aryl, and C₃ to C₁₂ heteroaryl.
1.37. A compound of any one of 1.1 to 1.11 and 1.17 to 1.34, wherein R¹ is 2-furyl.
1.38. A compound of any one of 1.1 to 1.11 and 1.17 to 1.34, wherein R¹ is -H.
1.39. A compound of any one of 1.1 to 1.6, 1.8 to 1.11 and 1.17 to 1.38, wherein the compound has the formula (2a) recited herein.
1.40. A compound of any one of 1.1 to 1.6, 1.8 to 1.11 and 1.17 to 1.38, wherein the compound has the formula (2a-i) recited herein.
1.41. A compound of any one of 1.1 to 1.6, 1.8 to 1.11 and 1.17 to 1.38, wherein the compound has the formula (2a-ii) recited herein.
1.43. A polymeric or co-polymeric compound comprising a repeating unit of formula (4) recited herein, wherein R is as defined in any one of 1.1 to 1.6.
1.44. A polymeric or co-polymeric compound according to 1.43, wherein x is defined as in 1.9 or 1.10.
1.45. A polymeric or co-polymeric compound according to any one of 1.43 to 1.44, wherein Y' = H.
1.46. A polymeric or co-polymeric compound according to any one of 1.43 to 1.45, wherein Z¹ = H.
1.47. A polymeric or co-polymeric compound according to any one of 1.43 to 1.46, wherein Y is as defined in any one of 1.23 to 1.28.
1.48. A polymeric or co-polymeric compound according to any one of 1.43 to 1.47, wherein Z is as defined in 1.29 to 1.34.
1.49. A polymeric or co-polymeric compound according to any one of 1.43 to 1.48, wherein R¹ is as defined in any one of 1.35 to 1.38.
1.50. A polymeric or co-polymeric compound according to any one of 1.43 to 1.49, wherein n is as defined in 1.18 or 1.20.
1.51. A polymeric or co-polymeric compound according to any one of 1.43 to 1.49, wherein m is as defined in 1.17 or 1.19.
1.52. A polymeric or co-polymeric compound according to any one of 1.43 to 1.51, wherein R¹⁰ is -H.
1.53. A polymeric or co-polymeric compound according to any one of 1.43 to 1.51, wherein R⁹ is selected from: -H, C₁ to C₁₀ alkyl, C₃ to C₁₂ cycloalkyl, C₂ to C₁₀ alkenyl, C₆ to C₁₂ aryl and C₃ to C₁₂ heteroaryl.
1.54. A polymeric or co-polymeric compound according to any one of 1.43 to 1.52, wherein R⁹ is -H.
1.55. A polymeric or co-polymeric compound according to any one of 1.43 to 1.52, wherein R⁹ is 2-furyl.
1.56. A polymeric or co-polymeric compound according to any one of 1.43 to 1.55, wherein P is from 3 to 40.
1.57. A polymeric or co-polymeric compound according to any one of 1.43 to 1.55, wherein P is from 4 to 20.
1.58. A polymeric or co-polymeric compound according to any one of 1.43 to 1.55, wherein P is from 5 to 15.
1.59. A polymeric or co-polymeric compound according to any one of 1.43 to 1.58, wherein the compound has the formula (4a) recited herein.
1.60. A polymeric or co-polymeric compound according to any one of 1.43 to 1.58, wherein the compound has the formula (4b) recited herein.
1.61. A polymeric or co-polymeric compound comprising a repeating unit of formula (5) recited herein, wherein R is defined as in any one of 1.1 to 1.6.
1.62. A polymeric or co-polymeric compound according to 1.61, wherein x is defined as in 1.8 to 1.10.
1.63. A polymeric or co-polymeric compound according to any one of 1.61 to 1.62, wherein Y' = H.
1.64. A polymeric or co-polymeric compound according to any one of 1.61 to 1.63, wherein Z¹ = H.
1.65. A polymeric or co-polymeric compound according to any one of 1.61 to 1.64, wherein Y is as defined in any one of 1.23 to 1.28.
1.66. A polymeric or co-polymeric compound according to any one of 1.61 to 1.65, wherein Z is as defined in 1.29 to 1.34.
1.67. A polymeric or co-polymeric compound according to any one of 1.61 to 1.66, wherein R¹ is as defined in any one of 1.35 to 1.38.
1.68. A polymeric or co-polymeric compound according to any one of 1.61 to 1.67, wherein n is as defined in 1.18 or 1.20.
1.69. A polymeric or co-polymeric compound according to any one of 1.61 to 1.67, wherein m is as defined in 1.17 or 1.19.
1.70. A polymeric or co-polymeric compound according to any one of 1.61 to 1.69, wherein P is from 3 to 1000.
1.71. A polymeric or co-polymeric compound according to any one of 1.61 to 1.69, wherein P is from 3 to 200.
1.72. A polymeric or co-polymeric compound according to any one of 1.61 to 1.69, wherein P is from 3 to 100.
1.73. A polymeric or co-polymeric compound according to any one of 1.61 to 1.69, wherein P is from 3 to 40.
1.74. A polymeric or co-polymeric compound according to any one of 1.61 to 1.69, wherein P is from 4 to 20.
1.75. A polymeric or co-polymeric compound according to any one of 1.61 to 1.69, wherein P is from 5 to 15.
1.76. A polymeric or co-polymeric compound according to any one of 1.61 to 1.75, wherein R⁴ is -Me, and R⁵ and R⁶ are -H.
1.77. A polymeric or co-polymeric compound according to any one of 1.61 to 1.75, wherein R⁴, R⁵ and R⁶ are -H.
1.78. A polymeric or co-polymeric compound according to any one of 1.61 to 1.77, wherein R¹¹ is a direct bond.
1.79. A polymeric or co-polymeric compound according to any one of 1.61 to 1.77, wherein R¹¹ is -OC(O)-.
1.80. A polymeric or co-polymeric compound according to any one of 1.61 to 1.79, wherein the compound has the formula (5a) recited herein.
1.81. A compound of any one of 1.1 to 1.11, 1.17 to 1.25 and 1.29 to 1.80, wherein at least one Y group comprises an amine.
1.82. A compound of any one of 1.1 to 1.11, 1.17 to 1.25 and 1.29 to 1.80, wherein at least one Y group comprises a primary or secondary amine.
1.83. A compound of any one of 1.1 to 1.11, 1.17, 1.19 to 1.25 and 1.29 to 1.80, wherein each Y group comprises an amine.
1.84. A compound of any one of 1.1 to 1.11, 1.17, 1.19 to 1.25 and 1.29 to 1.80, wherein each Y group comprises a primary or secondary amine.

### Methods Of Preparing Compounds of The Present Invention

In one aspect, the present invention provides a method of preparing an aromatic or heteroaromatic amide Mannich base for use as an epoxy resin curative and/or accelerator, said method comprising:
i) reacting an optionally substituted aromatic or heteroaromatic acid, or ester thereof, selected from hydroxy benzoic acid, hydroxypyridine carboxylic acid, pyrrole carboxylic acid, thiophene carboxylic acid, and furan carboxylic acid, or any ester thereof with a first polyamine having at least two nucleophilic amines, to form an aromatic or heteroaromatic amide;
ii) performing a Mannich reaction using the aromatic or heteroaromatic amide, an aldehyde, and a second amine having at least one nucleophilic amine.

As would be appreciated, at least one nucleophilic amine is required on the first polyamine and on the second amine in order for the amidation, and Mannich reactions to take place. The amidation consumes one of these nucleophilic amines by conversion into an amide, thus two nucleophilic amines are required on the first polyamine in order to yield a compound having a nucleophilic amino substituted amide. The second amine requires only one nucleophilic amine, in order for the Mannich reaction to take place. However, it is preferred that the second amine is a second polyamine, preferably a second polyamine having at least two nucleophilic amines, so that the Mannich amine group also bears at least one additional amine, preferably at least one nucleophilic amine.

This method is useful in preparing compounds having formula (2). The starting material is an aromatic or heteroaromatic core substituted with a carboxylic acid group, or ester thereof, selected from the group consisting of hydroxy benzoic acid, hydroxypyridine carboxylic acid, pyrrole carboxylic acid, thiophene carboxylic acid, and furan carboxylic acid, or an ester thereof, and which may be optionally substituted with one or more substituents. For example, the one or more substituents may independently have a structure defined by R, as defined hereinabove.

The aromatic or heteroaromatic core substituted with a carboxylic acid group, or ester thereof, is reacted with the first polyamine to form an aromatic or heteroaromatic amide.

The amidation reaction of the carboxylic acid group, or ester thereof, with the first polyamine would be within the capabilities of the skilled person.

A variety of aromatic or heteroaromatic carboxylic acids, and esters thereof are commercially available. Furthermore, conversion between the carboxylic acid and ester thereof is easily achievable by the skilled person, for example by esterification of the carboxylic acid with an alcohol, and by hydrolysis of the ester using acid or base.

The amidation reaction may proceed by heating with oxalic acid; by the use of amide coupling agents such as, HATU, HBTU, PyBOP, N,N'-Dicyclohexylcarbodiimide, carbonyldiimidazole; via halogenation of the carboxylic acid group to an acid halide, for example, using thionyl chloride, thionyl bromide, oxalyl chloride, phosphorus tribromide or phosphorus pentachloride; or via formation of an acid anhydride or carbamate. Various other methods of amidation are also known in the art, for example, M. M. Joullié and K. M. Lassen, Evolution of amide bond formation, *Arkivoc.,* 2010, (viii), 189-250.

Similarly, in the case of an aromatic or heteroaromatic core substituted with a carboxylic acid ester, this may be reacted with the first polyamine to form an aromatic or heteroaromatic amide. The amidation reaction of the carboxylic acid ester group with the first polyamine would be within the capabilities of the skilled person. Amidation of esters may proceed for example, by mixing the ester and amine and heating, preferably in the presence of a Lewis acid catalyst.

In the context of the present invention an "ester thereof" refers to the carboxylic acid in question with the acidic hydrogen replaced by a hydrocarbyl group.

The first polyamine may for example be an amine defined by the formula HN(Z)(Z¹), wherein Z and Z¹ are as defined in any of the embodiments described hereinabove.

The aromatic or heteroaromatic amide is then used in a Mannich reaction with an aldehyde the second amine. The Mannich reaction involves the reaction of the aromatic or heteroaromatic amide, the second amine and the aldehyde. Multiple synthetic routes are possible for reaction of these three components in forming the Mannich base, depending on the order of addition of the reactants. The skilled person is able to select a suitable stoichiometry of reagents depending on the synthetic route adopted. Typically the second amine and aldehyde would be present in molar excess relative to the aromatic or heteroaromatic amide, and stoichiometry may be readily modified to account for the presence of any residual aromatic or heteroaromatic carboxylic acid, or ester thereof, and also to account for the extent of the Mannich reactions desirable on the aromatic or heteroaromatic amide, i.e. the number of Mannich base groups to be added. An example stoichiometric ratio of second amine to aromatic or heteroaromatic amide is 2:1 to 20:1.

Optionally, preparing the Mannich base is achieved via the Betti reaction, which is a special form of the Mannich reaction, which comprises a condensation reaction between the second amine and the aldehyde, prior to reaction with aromatic or heteroaromatic amide. Condensation reaction between the second amine and the aldehyde provides an iminium ion which may then act as the electrophile in an electrophilic aromatic substitution reaction.

When the reaction between the aromatic or heteroaromatic amide and the second amine is performed in the presence of the aldehyde, the aldehyde may be added, either continuously over a period, or incrementally, to the reaction mixture. Alternatively or additionally, the second amine may be pre-reacted/pre-modified with the aldehyde, prior to contact with the aromatic or heteroaromatic amide. For instance, the second amine and the aldehyde can be pre-reacted to generate the corresponding iminium compound which may further react with the aromatic or heteroaromatic amide.

When the second amine is modified by a condensation reaction with the aldehyde, or reacted in the presence of the aldehyde in accordance with embodiments described herein, a typical molar ratio of the aldehyde to the second amine is from 0.1: 1 to 10: 1, preferably 0.5:1 to 5:1, more preferably from 1:1 to 3:1.

As part of the method, water that is formed from the condensation with the aldehyde is typically removed by distillation. Alternatively or additionally, the product can be further purified by distillation to further reduce the amount of free second amine and/or any added solvent.

The aldehyde may for example be an aldehyde defined by the formula H(CO)R¹, wherein R¹ is defined in any of the embodiments described hereinabove. In a preferred embodiment the aldehyde is formaldehyde or furfural.

The second amine may for example be an amine defined by the formula HN(Y)(Y¹), wherein Y and Y' are as defined in any of the embodiments described hereinabove.

In a preferred embodiment, the second amine is a second polyamine having at least one nucleophilic amine, preferably the second amine is a second polyamine having at least two nucleophilic amines, even more preferably the first and second polyamines are the same.

Suitable polyamines useful as the first polyamine, and/or the second amine, may be selected from: 1) an aliphatic primary di- or tri-amine; preferably an ether-group-containing aliphatic primary di- or tri-amine; 2) an aliphatic secondary amino-containing tri-amine having two primary aliphatic amino groups; 3) a polyamine having one or two secondary amino groups, preferably products of the reductive alkylation of primary aliphatic polyamines with aldehydes or ketones; or 4) an aromatic polyamine.

Suitable polyamines useful as the first polyamine, and/or the second amine, may be selected from: 2,2-dimethyl-1,3-propanediamine, 1,3-pentanediamine (DAMP), 1,5-pentanediamine, 1,5-diamino-2-methylpentane (MPMD), 2-butyl-2-ethyl-1, 5-pentanediamine (C11-nododiamine), 1,6-hexanediamine, 2,5-dimethyl-1,6-hexanediamine, 2,2 (4), 4-trimethylhexamethylenediamine (TMD), 1,7-heptanediamine, 1, 8-octanediamine, 1,9-nonanediamine, 1,10-decanediamine, 1,11-undecandiamine, 1,12-dodecanediamine, 1,2-,1,3- or 1,4-diaminocyclohexane, bis(4-aminocyclohexyl) methane (H 12-MDA), bis(4-amino-3-methylcyclohexyl) methane, bis(4-amino-3-ethylcyclohexyl) methane, bis(4-amino-3,5-dimethylcyclohexyl) methane, bis(4-amino-3-ethyl-5-methylcyclohexyl) methane, 1-amino-3-aminomethyl-3,5,5-trimethylcyclohexane (isophoronediamine or IPDA), 2- or 4-methyl-1,3-diaminocyclohexane or mixtures thereof, 1,3-bis(aminomethyl) cyclohexane, 1,4-bis(aminomethyl) cyclohexane, 2,5 (2,6)-bis(aminomethyl) bicyclo [2.2.1] heptane (NBDA), 3(4), 8(9)-Bis(aminomethyl) tricyclo [5.2.1.0^{2,6}] decane, 1,4-diamino-2,2,6-trimethylcyclohexane (TMCDA), 1,8-Me N-thandiamin, 3,9-bis(3-aminopropyl)-2,4,8,10-tetraoxaspiro [5.5] undecane, 1,3-bis(aminomethyl) benzene (MXDA), 1, 4-bis(aminomethyl) benzene, and combinations thereof; or wherein the polyamine is an aliphatic primary triamine selected from 4-aminomethyl-1,8-octanediamine, 1,3,5-tris(aminomethyl) benzene, 1,3,5-tris(aminomethyl) cyclohexane, tris(2-aminoethyl) amine, tris(2-amino-propyl) amine, tris(3-aminopropyl) amine and combinations thereof.

Suitable polyamines useful as the first polyamine, and/or the second amine, may be ether-group-containing aliphatic primary di-amines. Suitable ether-group-containing aliphatic primary di-amines include those selected from: bis (2-aminoethyl) ether, 3,6-dioxaoctane-1,8-diamine, 4,7-dioxadecane-1, 10 diamine, 4,7-dioxadecane-2,9-diamine, 4,9-dioxadodecane-1,12-diamine, 5,8-dioxadodecane-3,10-diamine, 4,7,10-trioxatridecan-1,13-diamine, or oligomers of any of the foregoing; polytetrahydrofurandiamines, such as bis(3-aminopropyl) polytetrahydrofurans, cycloaliphatic diamines containing ether groups preferably derived from propoxylation and subsequent amination of 1,4-dimethylol cyclohexane (for example, available as Jeffamine ^{®} particular RFD 270 (Huntsman)), and polyoxyalkylenediamines, such as polyoxypropylenediamines, preferably derived from amination of polyoxyalkylenediols, for example, those available under the name Jeffamine ^{®} (from Huntsman), under the name polyetheramine (from BASF) or under the name PC amine ^{®} (from Nitroil). In particular, suitable polyoxyalkylene di- or triamines are Jeffamine ^{®} D-230, Jeffamine ^{®} D-400, Jeffamine ^{®} D-2000, Jeffamine ^{®} EDR-104, Jeffamine ^{®} EDR-148, Jeffamine ^{®} EDR-176, Jeffamine ^{®} T-403, Jeffamine ^{®} T-3000, Jeffamine ^{®} T-5000, or the corresponding amines by BASF or Nitroil.

Suitable polyamines useful as the first polyamine, and/or the second amine, may be ether-group-containing aliphatic primary tri-amines. Suitable ether-group-containing aliphatic primary tri-amines include polyoxyalkylenetriamines, preferably derived from amination of polyoxyalkylenetriols.

Suitable polyamines useful as the first polyamine, and/or the second amine, may be secondary amino-containing tri-amines. Suitable aliphatic secondary amino-containing tri-amines having two primary aliphatic amino groups include: 3-(2-aminoethyl) aminopropylamine, bis(hexamethylene) triamine (BHMT), diethylenetriamine (DETA), triethylenetetramine (TETA), tetraethylenepentamine (TEPA), pentaethylenehexamine (PEHA) or higher homologs of linear polyethyleneamines such as polyethylenepolyamine with 5 to 7 ethylenepolyamine units (HEPA), products of the multiple cyanoethylation or cyanobutylation and subsequent hydrogenation of primary polyamines having at least two primary amino groups, such as Dipropylenetriamine (DPTA), N-(2-aminoethyl)-1,3-propanediamine (N3-amine), N,N'-bis(3-aminopropyl) ethylenediamine (N4-amine), N,N'-bis (3-aminopropyl)-1,4-diaminobutane, N5-(3-aminopropyl)-2-methyl-1,5-pentanediamine, N3-(3-aminopentyl)-1,3-pentanediamine, N5-(3-Amino-1-ethyl-propyl)-2-methyl-1,5-pentanediamine, N,N'-bis (3-amino-1-ethylpropyl)-2-methyl-1,5-pentanediamine, and combinations thereof.

Suitable polyamines useful as the first polyamine, and/or the second amine, may be polyamines having one or two secondary amino groups. Suitable polyamines having one or two secondary amino groups include: N¹-benzyl-1,2-propanediamine, N¹-(4-methoxybenzyl)-1,2-propanediamine, N-benzyl-1,3-bis (aminomethyl) benzene, N,N'-Dibenzyl-1,3-bis (aminomethyl) benzene, N-2-ethylhexyl-1,3-bis (aminonyl) benzene, N,N'-bis(2-ethylhexyl)-1,3-bis(aminomethyl) benzene, and partially styrenated polyamines, such as partially styrenated 1,3-bis(aminomethyl) benzene (MXDA) (available from Mitsubishi gas Chemical).

Suitable polyamines useful as the first polyamine, and/or the second amine, may be aromatic polyamines. Suitable aromatic polyamines include m- and p-phenylenediamine, 4,4'-, 2,4'- and/or 2,2'-diaminodiphenylmethane, 3,3'-dichloro-4,4'-diaminodiphenylmethane (MOCA) diisocyanate, 2,4- and / or 2,6-toluene diamine (available as Ethacure 300 from Albemarle^{®}), mixtures of 3,5-dimethylthio-2,4- and -2,6-toluene diamine, mixtures of 3,5-diethyl -2,4- and -2,6-toluylenediamine (DETDA), 3,3',5,5'-tetraethyl-4,4'-diaminodiphenylmethane (M-DEA), 3,3',5,5'-tetraethyl-2,2'-dichloro-4,4'-diaminodiphenylmethane (M-CDEA), 3,3'-diisopropyl-5,5'-dimethyl-4,4'-diaminodiphenylmethane (M-MIPA), 3,3',5,5'-tetraisopropyl-4,4'-diaminodiphenylmethane (M-DIPA), 4,4'-diamino diphenylsulfone (DDS), 4-amino-N-(4-aminophenyl) benzenesulfonamide, 5,5'-methylenedianthranilic acid, dimethyl (5,5'-methylenedithethranilate), 1,3-propylenebis(4-aminobenzoate), 1,4-butylenebis(4-aminobenzoate), polytetramethyleneoxide-bis(4-aminobenzoate), 1,2-bis(2-aminophenylthio)ethane, 2-methylpropyl (4-chloro-3,5-diaminobenzoate), t-Butyl (4-chloro-3,5-diaminobenzoate), and combinations thereof.

The amines described above can also be adducted with epoxides or epoxy resins and further modification may be possible with fatty acids to form amido or polyamides.

Optionally, said polyamine further comprises amido or polyamide functional groups derived from adduction of the polyamine with one or more epoxides and/or modification with one or more fatty acids.

The compounds of the present invention may comprise one or more Y groups, one or more Z groups, or one or more pair of adjacent of Y and Y' groups, or one or more pair of adjacent Z and Z¹ groups, derived from any of the above listed amines. As would be appreciated, a pair of adjacent Y and Y' groups, or Z and Z¹ groups may be derived from a single polyamine by reaction of a secondary amine having the formula HNYY¹ or HNZZ¹, in the Mannich reaction, or amidation reaction, respectively. Where a primary polyamine reacts in the Mannich reaction, or amidation reaction, the resulting Y' or Z¹ group will be hydrogen.

In another aspect, the present invention provides a method of preparing a polymeric hydroxy benzamide Mannich base for use as an epoxy resin curative and/or accelerator, said method comprising:
i) performing a polycondensation reaction with an optionally substituted hydroxy benzoic acid, or ester thereof; and a first aldehyde, a ketone, or hexamine to give a polymeric hydroxy benzoic acid, or ester thereof;
ii) reacting the polymeric hydroxy benzoic acid, or ester thereof, with a first polyamine having at least two nucleophilic amines, to give a polymeric hydroxy benzamide;
iii) performing a Mannich reaction using the polymeric hydroxy benzamide, a second aldehyde, and a second amine having at least one nucleophilic amine.

In a preferred embodiment, the second amine is a polyamine having at least one nucleophilic amine, more preferably the second amine is a polyamine having at least two nucleophilic amines.

This method is useful in preparing polymeric compounds having formula (4). The starting material is a hydroxy benzoic acid, or ester thereof, which may be optionally substituted with one or more substituents. For example, the one or more substituents may independently have a structure defined by R, as defined hereinabove. The hydroxy benzoic acid, or ester thereof, is reacted with a first aldehyde, a ketone, or hexamine in a polycondensation reaction. The polycondensation reaction forms a novolak type polymer comprising hydroxy benzamide moieties linked by a single carbon linker. As would be appreciated, the single carbon linker will either be unsubstituted, i.e. substituted with two hydrogens, or substituted with one or two substituents defined by the aldehyde or ketone used in the polycondensation reaction. The use of formaldehyde or hexamine in the polycondensation reaction will provide an unsubstituted single carbon linker. In a preferred embodiment, the single carbon linker is defined by L, as defined hereinabove.

Polycondensation reactions are known in the art and are widely used for the production of novolak type polymers from phenolic compounds and aldehydes or ketones. Hexamine may also be used in this step.

In a preferred embodiment, the first aldehyde is formaldehyde or furfural. In a preferred embodiment, the second aldehyde is formaldehyde or furfural. In a preferred embodiment, the first and second aldehydes are the same.

The polymerised hydroxy benzoic acid, or ester thereof, is then reacted with the first polyamine to form a polymeric hydroxyl benzamide. The amidation reaction may occur in much the same way as the amidation reaction of a monomeric aromatic or heteroaromatic carboxylic acid, or ester thereof as described hereinabove.

The polymerised hydroxy benzamide is then reacted with the second amine and second aldehyde to form a polymeric hydroxyl benzamide Mannich base. The Mannich reaction may occur in much the same way as the Mannich reaction of a monomeric aromatic or heteroaromatic amide as described hereinabove.

In a preferred embodiment, the polycondensation reaction in step i) is catalysed using acid-catalysis, such as sulfuric acid, oxalic acid, hydrochloric acid or sulfonic acids, preferably wherein the aldehyde is formaldehyde.

In another aspect, the present invention provides a method of preparing a polymeric hydroxy benzamide Mannich base for use as an epoxy resin curative and/or accelerator, said method comprising:
i) performing a vinyl polymerisation reaction with optionally substituted and alkenyl substituted hydroxy benzoic acid, or ester thereof, to give a polymeric hydroxy benzoic acid, or ester thereof;
ii) reacting the polymeric hydroxy benzoic acid, or ester thereof, with a first polyamine having at least two nucleophilic amines, to give a polymeric hydroxy benzamide;
iii) performing a Mannich reaction using the polymeric hydroxy benzamide, an aldehyde, and a second amine having at least one nucleophilic amine.

In a preferred embodiment, the second amine is a polyamine having at least one nucleophilic amine, more preferably the second amine is a polyamine having at least two nucleophilic amines.

This method is useful in preparing polymeric compounds having formula (5). The starting material is a vinyl substituted hydroxy benzoic acid, or ester thereof, which may be optionally substituted with one or more further substituents. For example, the one or more further substituents may independently have a structure defined by R, as defined hereinabove. The vinyl substituted hydroxy benzoic acid, or ester thereof, is polymerised using a vinyl polymerisation reaction. The vinyl polymerisation reaction forms an alkane chain backbone. The alkane chain backbone may be unsubstituted, i.e. be bonded to three hydrogen atoms per repeating unit, or substituted with one to three substituents per repeating unit. Similarly, the benzene ring may be directly bonded to the alkane chain backbone, or bonded by another group, as defined hereinbelow. As would be appreciated, the substituents of the alkane chain backbone are determined by substituents present on the vinyl group prior to polymerisation. In a preferred embodiment, the alkane chain backbone may be unsubstituted, by three groups per repeating unit, wherein the groups are defined as R⁴, R⁵ and R⁶ as defined hereinabove.

Vinyl polymerisation is well known in the art and is used for preparation of various materials such as polyvinyl chloride, polyacrylonitrile, and polystyrene. The polymerisation of vinyl monomers is within the capabilities of the skilled person.

In a preferred embodiment, the vinyl polymerisation reaction in step i) is one of:
free radical polymerisation, preferably initiated with a radical initiator, such as a dihalogen, AIBN, ABCN, di-tert-butyl peroxide, benzyl peroxide, peroxydisulfate salts or a transition metal catalyst;
anionic polymerisation, preferably initiated with an organometallic base, such a BuLi;
cationic polymerisation, preferably initiated with a protic acid or Lewis acid; or
metallocene catalysis, preferably Ziegler-Natta catalysis.

In a preferred embodiment, the method further comprises the step of vinylating an optionally substituted hydroxy benzoic acid, or ester thereof, to provide the vinyl hydroxy benzoic acid, or ester thereof, preferably wherein the vinylation reaction is a Heck reaction, Suzuki reaction, Negishi coupling, Kumada coupling, or cross dehydrogenative coupling.

In some embodiments, any one of the above described methods of preparing a compound of the present invention may further comprise the subsequent steps of:
i) reacting a dimer acid with a nucleophilic amine group present on the aromatic or heteroaromatic Mannich base or polymeric hydroxy benzamide Mannich base, to form an amide; and
ii) reacting a third polyamine having at least two nucleophilic amines, with the dimer acid to form a second amide.

As would be appreciated, dimer acids have two carboxylic acid groups, and as such, the first carboxylic acid group is reacted in step i) and the second carboxylic acid group reacted in step ii).

### Epoxy Resin Curatives

In another aspect, the present invention relates to epoxy resin curative composition comprising a compound as described herein.

It has been found that the epoxy resin curative composition may usefully comprise further amine compounds in order to adjust its properties to adapt the composition to a desired purpose. For example, properties such as the viscosity or active hydrogen equivalent weight may be adjusted by the presence of one or more further amine compounds. It has also been usefully found that excess amine can be used in the amidation and/or Mannich reaction steps of the preparation of the compounds of the present invention and then unreacted amine can be retained to adjust properties of the epoxy resin curative composition without the need for separation. This allows for a more efficient one pot preparation of such compositions. Nevertheless, separation of any excess amine used in the amidation and/or Mannich reaction steps could be easily achieved by the skilled person, if desired.

In some embodiments, the epoxy resin curative composition comprises further amine compounds. Preferably, the epoxy resin curative composition further comprises an amine compound having the formula HN(Y)(Y¹), wherein Y and Y' are as defined herein and are the same as the Y and Y' groups, respectively, which are present as amine substituents in the amido-amine compound also comprised within the composition, i.e. wherein the epoxy resin curative composition further comprises an amine compound wherein the amine compound is the amine from which at least one of the Mannich amine groups is derived.

In some embodiments, the epoxy resin curative composition further comprises an amine compound having the formula HN(Z)(Z¹), wherein Z and Z¹ are as defined herein and are the same as Z and Z¹ groups, respectively, in the amido-amine compound also comprised within the composition, i.e. wherein the epoxy resin curative composition further comprises an amine compound wherein the amine compound is the amine from which at least one of the amino substituted amide groups is derived.

In some embodiments, the epoxy resin curative composition further comprises an amine compound having the formula HN(Y)(Y¹), wherein Y and Y' are as defined herein and are the same as Y and Y' groups, respectively, which are present as amine substituents in the amido-amine compound also comprised within the composition, and an amine compound having the formula HN(Z)(Z¹), wherein Z and Z¹ are as defined herein and are the same as the Z and Z¹ groups, respectively, are as defined in said amido-amine compound also comprised within the composition.

The epoxy resin curative composition may optionally further comprise any amine compound described herein as suitable for use as the first polyamine, and/or the second amine of the method steps described herein.

Any suitable organic solvent may be used as a solvent or diluent in a resin composition of the present invention, for example MeCN, benzene, methanol, ethanol, IPA, butanol, chloroform, DCM, diethyl ether, DMF, dioxane, ethyl acetate, petroleum ether, kerosine, pentane, hexane, heptane, MTBE, NMP, THF, toluene, xylene and mixtures thereof. Toluene is a particularly suitable solvent or diluent for salicylamide Mannich base compounds and resins thereof of the present invention. As would be appreciated, methods of forming and handling a resin composition from a given compound would be known by the skilled person.

In preferred embodiments, the epoxy resin curative composition comprises less than 0.1 wt.% of free aromatic carboxylic acid, or ester thereof, such as salicylic acid. More preferably, the composition comprises less than 100 ppm, even more preferably less than 50 ppm, of free aromatic carboxylic acid, or ester thereof. The content of free aromatic carboxylic acid, or ester thereof may be reduced, or substantially eliminated, in the curative compositions according to the present invention through the use of an excess of first polyamine, second amine, and/or the aldehyde where the aromatic carboxylic acid, or ester thereof is employed and/or through purification techniques, for instance distillation.

In yet another aspect, the present invention provides a method for preparing a cured epoxy resin, said method comprising:
a) contacting an epoxy resin with a compound as described herein; and
b) forming a cured epoxy resin, preferably wherein the epoxy resin is selected from glycidyl amines, epoxidized novolacs and bisphenols (A or F) or halogenated analogues thereof.

Examples of epoxy-based resins suitable for use in the present invention include polyglycidyl ethers of polyhydric phenols, epoxidised novolacs or similar glycidated polyphenolic resins, glycidated bisphenols, such as glycidated bisphenol A or F, or halogenated (e.g. chlorinated or fluorinated) analogues thereof; polyglycidyl ethers of alcohols, glycols or polyglycols, and polyglycidyl esters of polycarboxylic acids. Preferred examples of epoxy resins are polyglycidyl ethers of a polyhydric phenol. Polyglycidyl ethers of polyhydric phenols can be produced, for example, by reacting an epihalohydrin with a polyhydric phenol in the presence of an alkali. Examples of suitable polyhydric phenols include: 2,2-bis (4-hydroxyphenyl) propane (bisphenol-A); 2,2-bis(4-hydroxy-3-tert-butylphenyl)propane; 1,1-bis(4-hydroxyphenyl) ethane; 1,1-bis(4-hydroxyphenyl) isobutane; bis(2-hydroxy-1-naphthyl) methane; 1,5-dihydroxynaphthalene; 1,1-bis(4-hydroxy-3-alkylphenyl) ethane and the like. Commercial examples of preferred epoxy resins that may be used include EPILOK 60-600 (RTM).

The epoxy resin to which the curative of the present invention is added may include other additives, such as flow control additives, antifoam agents, or anti-sag agents, as well as other additives such as pigments, reinforcing agents, fillers, elastomers, stabilizers, extenders, plasticizers, or flame retardants depending on the application.

The epoxy resin can be any epoxy resin which can be cured by the compound of the present invention. Generally, the epoxy resin can be any curable epoxy resin and may have, for instance, a 1,2-epoxy equivalency greater than one and preferably, on average, more than 1.5 epoxide groups per molecule. The epoxy resin can be saturated or unsaturated, linear or branched, aliphatic, cycloaliphatic, aromatic or heterocyclic, and may be substituted, provided such substituents do not interfere with the curing reaction. Such substituents can include bromine.

The epoxy resin to which the compound of the present invention is added may include other additives, such as flow control additives, antifoam agents, or anti-sag agents, as well as other additives such as pigments, reinforcing agents, fillers, elastomers, stabilizers, extenders, plasticizers, or flame retardants depending on the application.

In a final aspect, the present invention provides a use of a compound as described herein for causing the crosslinking of an epoxy resin.

The invention will now be described by reference to the following non-limiting Examples and the Figures.

### EXAMPLES

### EXAMPLE 1 - Preparation of an Epoxy Resin Curative Composition Comprising Mannich Base A according to Formula (I)

The intermediate compound, salicylamide A (shown below) was prepared by reaction of salicylic acid and triethylene tetramine.

730 grams (5 mols) of triethylene tetramine was added to a 1 litre, round-bottomed flask equipped with a condenser, mechanical stirrer, addition funnel and thermometer.

9 grams (0.1 mol) of oxalic acid was added and the materials mixed. The flask was set for atmospheric reflux, agitator started and gently heated to 50°C.

At 50°C, 138 grams (1 mol) of salicylic acid was added in 4 aliquots over a 45-minute period, using cooling water to control the exotherm and maintain the temperature between 50 to 75°C throughout the addition. Once fully added, the flask was set for atmospheric distillation and a nitrogen blanket applied. The material was heated to 230°C removing reaction water during the heat up and measuring the extent of the reaction by acid value. The process was continued until the acid value fell below 2 mg/KOH/gm. The reaction mass was cooled before continuing to the secondary stage.

A sample of the resulting reaction mass was tested for its properties as an epoxy resin curative. Said reaction mass is approximately a 1:4 molar mixture of Salicylamide A : triethylene tetramine.

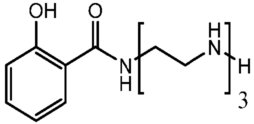

### Salicylamide A

**Table 1. Properties of the epoxy resin curative composition comprising salicylamide A.**

| | Characteristic | Method | Result |
|---|---|---|---|
| | Colour | ASTM D6166 | 12 |
| | Viscosity at 25°C | ASTM D2556-14(2018) | 140 m·Pas |
| | Acid value | ASTM D974-14e2 | 1.29 mg/KOH/gm |
| | Free salicylic acid | Calculated | 0.3% |
| | Active hydrogen equivalent weight | Calculated | 30 |
| | Reactivity * | Experimental | 12.4 Minutes |

Salicylamide A is a low viscosity amber liquid with good clarity. This material was tested for gel-time reactivity and exhibited outstanding cure rate. <15-minute gel-time in 150 g mass.

An epoxy resin curative composition comprising salicylamide Mannich base A (shown below) was prepared by reaction of salicylamide A and triethylene tetramine.

Salicylamide A [850 grams, 1 mol of salicylamide A in 4 mol excess of triethylene tetramine] was added to a 1 litre, round-bottomed flask equipped with a condenser, mechanical stirrer, addition funnel and thermometer.

The material was equilibrated at 25 to 30°C and the flask was set for atmospheric reflux. The agitator started and 148 grams [1.8 mols] of an aqueous solution of 36.5% formaldehyde was added over a 30-minute period controlling using cooling water to control the exotherm and maintain the temperature below 90°C throughout the addition. Once fully added, the flask was set for atmospheric reflux and held between 85 to 90°C monitoring the viscosity at 25°C using a cone and plate viscometer. Following a hold of 210 minutes at 85-90°C the viscosity increased to 600 m·Pas. The flask was then set for atmospheric distillation and heated over a 90-minute period to 120°C. Once at 120°C the flask was configured for vacuum distillation and vacuum distilled at 120°C under 88,046 to 94,819 Pa for 30 minutes collecting any residual distillate, before being cooled to provide an epoxy resin curative composition comprising salicylamide Mannich Base A.

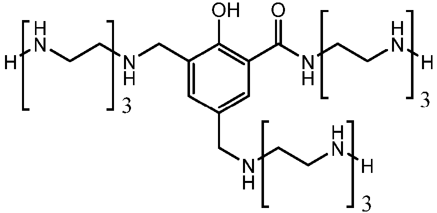

### Salicylamide Mannich Base A

**Table 2. Properties of the epoxy resin curative composition comprising salicylamide Mannich base A.**

| | Characteristic | Method | Result |
|---|---|---|---|
| | Colour | ASTM D6166 | 14 |
| | Viscosity at 25°c | ASTM D2556-14(2018) | 1130 m·Pas |
| | Active hydrogen equivalent weight | Calculated | 41 |
| | Reactivity * | Experimental | 17.3 Minutes |

| | | | |
|---|---|---|---|
| *Reactivity undertaken with an epoxy resin with epoxy equivalent weight (EEW) 190 and a mix ratio of 100:20 by weight. | | | |

### EXAMPLE 2 - Preparation of an Epoxy Resin Curative Composition Comprising Mannich Base B according to Formula (I)

The intermediate compound, salicylamide B (shown below) was prepared by reaction of salicylic acid and Jeffamine D230 (RTM).

920 grams (4 mols) of Jeffamine D230 (RTM) was added to a 2 litre, round-bottomed flask equipped with a condenser, mechanical stirrer, addition funnel and thermometer.

18 grams (0.2 mol) of oxalic acid was added and the materials mixed together. The flask was set for atmospheric reflux, agitator started and gently heated to 50°C.

At 50°C, 138 grams (1 mol) of salicylic acid was added and once fully added, the flask was set for atmospheric distillation and a nitrogen blanket applied. The material was heated to 230°C removing reaction water during the heat up and measuring the extent of the reaction by acid value. The process was continued until the acid value fell below 2 mg/KOH/gm. The salicylamide was cooled and 146 grams [1 mol] of triethylene tetramine added.

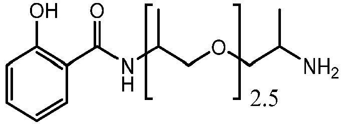

### Salicylamide B

**Table 3. Properties of the epoxy resin curative comprising salicylamide B.**

| | Characteristic | Method | Result |
|---|---|---|---|
| | Colour | ASTM D6166 | 7 |
| | Viscosity at 25°c | ASTM D2556-14(2018) | 45 m·Pas |
| | Acid value | ASTM D974-14e2 | 1.53 mg/KOH/gm |
| | Free salicylic acid | Calculated | <1% |
| | Active hydrogen equivalent weight | Calculated | 64 |
| | Reactivity * | Experimental | 205 Minutes |

| | | | |
|---|---|---|---|
| *Reactivity undertaken with an epoxy resin with EEW 190 and a mix ratio of 100:32 by weight. | | | |

An epoxy resin curative composition comprising, salicylamide Mannich base B (shown below) was prepared by *in situ* reaction of salicylamide B prepared as described above, with triethylene tetramine.

920 grams (4 mols) of Jeffamine D230 (RTM) was added to a 2 litre, round-bottomed flask equipped with a condenser, mechanical stirrer, addition funnel and thermometer.

18 grams (0.2 mol) of oxalic acid was added and the materials mixed together. The flask was set for atmospheric reflux, agitator started and gently heated to 50°C.

At 50°C, 138 grams (1 mol) of salicylic acid was added and once fully added, the flask was set for atmospheric distillation and a nitrogen blanket applied. The material was heated to 230°C removing reaction water during the heat up and measuring the extent of the reaction by acid value. The process was continued until the acid value fell below 2 mg/KOH/gm. The reaction mass was cooled before adding 146 grams triethylene tetramine (1mol).

The mixture was then equilibrated at 50°C and 164 grams (2 mol) of 36.5% aqueous formaldehyde solution was added slowly over a 30-minute period controlling using cooling water to control the exotherm and maintain the temperature below 90°C throughout the addition. Once fully added, the flask was set for atmospheric reflux and held at reflux between 99-101°C for 120 minutes. Following a hold of 120 minutes at reflux the flask was then set for atmospheric distillation and heated over a 90-minute period to 120°C. Once at 120°C the flask was configured for vacuum distillation and vacuum distilled at 120°C under 88046 to 94819 Pa for 30 minutes collecting any residual distillate before being cooled to provide an epoxy resin curative composition comprising salicylamide Mannich Base B which comprises a number of compounds within the scope of formula (2). As would be appreciated, the combination of amines present during the Mannich reaction step would result in a combination of Mannich reaction product as shown below.

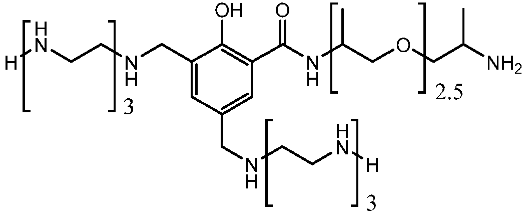

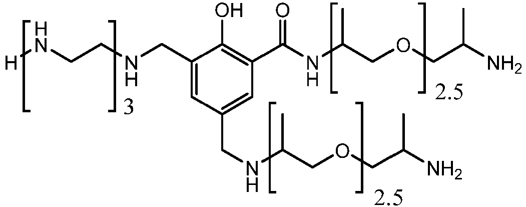

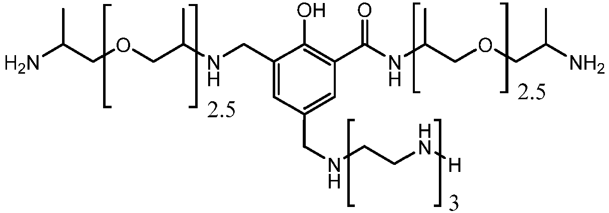

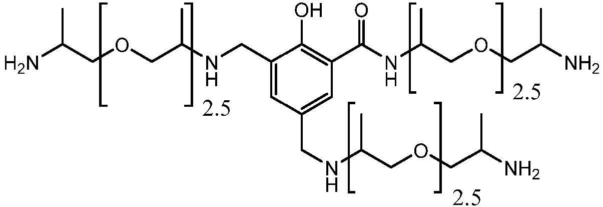

Salicylamide Mannich Base Compounds Present in Salicylamide Mannich Base B

**Table 4. Properties of epoxy resin curative composition comprising salicylamide Mannich base B.**

| | Characteristic | Method | Result |
|---|---|---|---|
| | Colour | ASTM D6166 | 9 |
| | Viscosity at 25°c | ASTM D2556-14(2018) | 98 m·Pas |
| | Active hydrogen equivalent weight | Calculated | 41 |
| | Reactivity * | Experimental | 472 Minutes |

| | | | |
|---|---|---|---|
| *Reactivity undertaken with an epoxy resin with EEW 190 and a mix ratio of 100:22 by weight. | | | |

### EXAMPLE 3 - Comparative Epoxy Resin Curative Composition Comprising a Phenolic Mannich Base

As another comparative example, comparative data is provided for a standard known phenolic Mannich base. Curamine 31-537 (RTM) is a commercially available phenolic Mannich base [Bitrez Ltd] preparable by Mannich reaction of phenol, formaldehyde and triethylene tetramine.

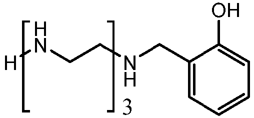

### Curamine 31-537 (RTM)

**Table 5. Comparative Phenolic Mannich Base Properties.**

| | Characteristic | Method | Result |
|---|---|---|---|
| | Viscosity at 25°c | ASTM D2556-14(2018) | 900 m·Pas |
| | Active hydrogen equivalent weight | Calculated | 50 |
| | Reactivity * | Experimental | 12.0 Minutes |

| | | | |
|---|---|---|---|
| *Reactivity undertaken with an epoxy resin with EEW 190 and a mix ratio of 100:25 by weight. | | | |

The compounds of the present invention are also able to provide comparable properties, such as reactivity, to more conventional phenolic Mannich base hardeners, whilst avoiding the use of phenol and making use of innocuous and/or biobased carboxylic acid or ester starting materials.

### EXAMPLE 4 - Preparation of Mannich Base C According to Formula (9)

The dimeric compound, salicylamide Mannich base C (shown below) was prepared by reaction of salicylic acid, triethylene tetramine and a dimer acid.

292 grams (2 mols) of triethylene tetramine was added to a 1 litre, round-bottomed flask equipped with a condenser, mechanical stirrer, addition funnel and thermometer.

1.54 grams (0.1 mol) of oxalic acid was added and the materials mixed. The flask was set for atmospheric reflux, agitator started and gently heated to 50°C.

At 50°C, 276 grams (2 mols) of salicylic acid was added in 4 aliquots over a 60-minute period, using cooling water to control the exotherm and maintain the temperature between 50 to 75°C throughout the addition. Once fully added, the flask was set for atmospheric distillation and a nitrogen blanket applied. The material was heated to 230°C removing reaction water during the heat up and measuring the extent of the reaction by acid value. The process was continued until the acid value fell below 2 mg/KOH/gm. The reaction mass was cooled to 100°C and transferred to a 3 litre, round-bottomed flask equipped with a condenser, mechanical stirrer, addition funnel and thermometer. 564 grams (1 mol) of dimer acid was added to the flask followed by 1.54 grams (0.1 mol) of oxalic acid. The flask was set for atmospheric azeotropic distillation and heated to 150°C. Distillate was received from 150°C and the temperature increased to 230°C. Once at 230°C the product was held for 30 minutes then cooled to 85°C and 584 grams (4 mols) of triethylene tetramine added. 400 grams of xylene was added. The flask was set for atmospheric reflux and the temperature was adjusted to 25°C. 132 grams of 91-93% paraformaldehyde [4 mols] was added over a 30-minute period maintaining the temperature below 50°C. The material was then mixed at 50°C for 60 minutes before heating to 80°C over a 45-minute period. Once at 80°C the material was held for 2 hours. The flask was configured for atmospheric azeotrope and heated to 130°C recovering distillate over a 3-hour period. The product was then cooled to provide salicylamide Mannich Base C.

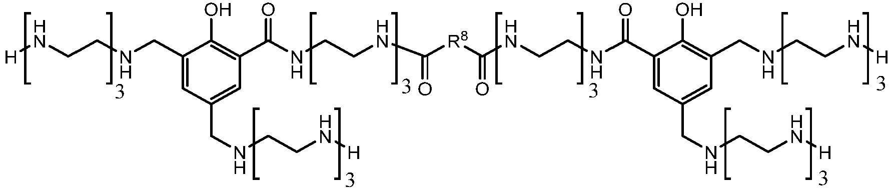

### Salicylamide Mannich Base C

In this example, R⁸ is a C₃₄ alkyl, cycloalkyl, alkenyl, or cycloalkenyl group derived from the C₃₆ dimer acid.

**Table 6. Properties of Salicylamide Mannich Base C.**

| | Characteristic | Method | Result |
|---|---|---|---|
| | Appearance | Visual | Viscous liquid |
| | Colour | ASTM D6166 | 16 |
| | Viscosity at 25°C | ASTM D2556-14(2018) | 21,400 m·Pas |

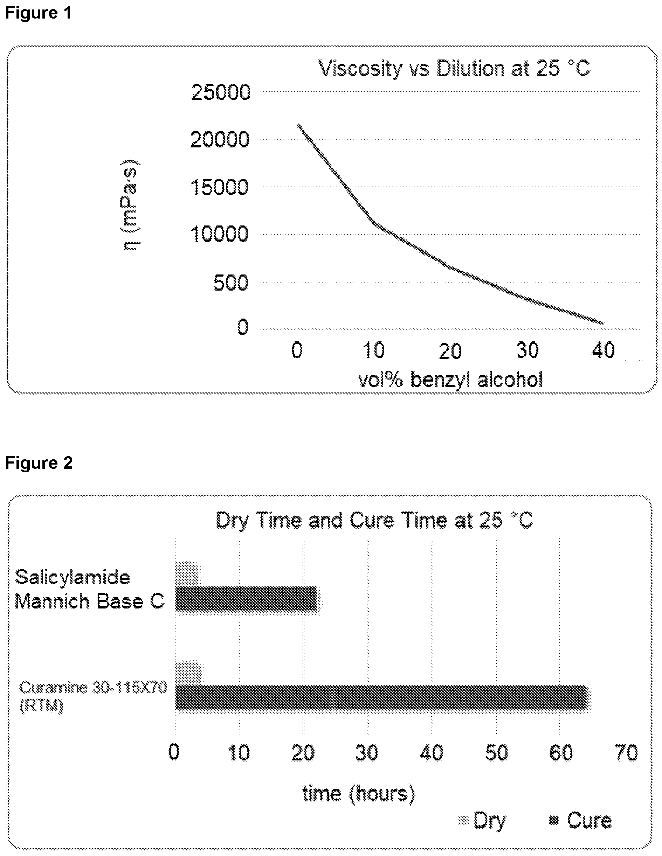

This material was then diluted with benzyl alcohol in order to reduce the viscosity. The relationship between the viscosity (as measured by ASTM D2556-14(2018)) and the vol% dilution with benzyl alcohol is shown in Figure 1. As can be seen, even a relatively minor level of dilution led to a major decrease in viscosity. The properties of a 63:37 vol/vol mixture of salicylamide Mannich base C : benzyl alcohol are shown below in Table 7.

**Table 7. Properties of salicylamide Mannich Base C Diluted with Benzyl Alcohol in a 63:37 vol/vol ratio.**

| | Characteristic | Method | Result |
|---|---|---|---|
| | Appearance | Visual | Clear dark liquid |
| | Colour | ASTM D6166 | 16 |
| | Viscosity at 25°C | ASTM D2556-14(2018) | 875 m·Pas |

Figure 2 demonstrates the thin film dry times and cure times of salicylamide Mannich base C diluted in with xylene as compared to those of Curamine 30-115X70 (RTM), a known industry standard curative. As can be seen, salicylamide Mannich base C enjoys more rapid dry times and cure times than those of those of Curamine 30-115X70 (RTM).

### EXAMPLE 5 - Oestrogenic Activity Testing of Salicylamide Mannich Base A

It is well known that bisphenol A is an agonist for the oestrogen receptor which leads to toxicity in humans and in the environment. The use of bisphenol A in epoxy systems is often avoided for this reason. Various phenolic compounds, such as phenolic Mannich bases may also pose a risk of acting as oestrogen agonists, this is thought to be due to structural similarity between the phenolic group and the phenolic group present in oestrogen itself.

Salicylamide Mannich base A was tested for activity at the oestrogen receptor, along with bisphenol A and 17β-oestradiol (also referred to as E2), as controls. The tests were performed using MELN cells [hER-stably transfected model of the human breast cancer MCF-7 cells]. The use of MELN cells for screening cytotoxicity and oestrogenic activity is a standard test known in the art, for example, as described in Berckmans P, Leppens H, Vangenechten C, Witters H., "Screening of endocrine disrupting chemicals with MELN cells, an ER-transactivation assay combined with cytotoxicity assessment", Toxicol In Vitro. 2007, 21(7), 1262-7.

**Table 8. Oestrogenic Activity Testing Using MELN Cells.**

| Compound | Structure | Cytotoxicity | Agonist response | Log EC₅₀ |
|---|---|---|---|---|
| 17β-oestradiol | 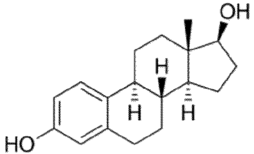 | No | Positive | -10.71 |
| Bisphenol A | 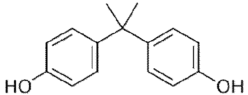 | No | Positive | -6.44 |
| Salicylamide Mannich Base A | 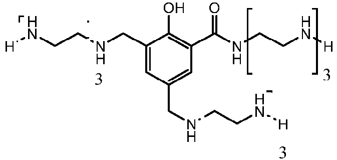 | No | Negative | N/A |

As can be seen from these results, salicylamide Mannich base A presented neither any cytotoxicity nor any activity at the oestrogen receptor. Bisphenol A and 17β-oestradiol presented oestrogenic activity as expected.

This shows that in spite of the phenolic moiety, salicylamide Mannich base A does not appear to present any risk of toxicity via oestrogen agonism. This suggests that the broader class of amido-amine hardeners carries a reduced risk of oestrogen agonism.

## Claims

1. A compound of formula (2): 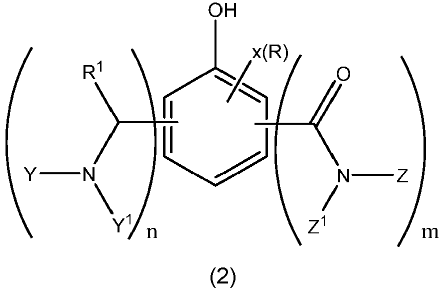 wherein:
each R group is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkyloxy, C₁ to C₂₀ alkylamino, -OH, -OR², -NH₂, -NHR², - NR²₂, -C(O)OH, -C(O)OR², -C(O)NH₂, -C(O)NHR², -C(O)NR²₂, -O(CO)H, -O(CO)R², -NH(CO)H, -NH(CO)R², -NR²(CO)H, -NR²(CO)R², -SH, -SR², -SO₂H, -SO₂R², - SO₃R², -SO₃H, -SiR²₃, -NO₂, -CN, -F, -Cl, -Br, and -I;
each R¹ group is independently selected from: -H, C₁ to C₁₀ alkyl, C₁ to C₁₀ haloalkyl, C₃ to C₁₂ cycloalkyl, C₂ to C₁₀ alkenyl, C₂ to C₁₀ alkynyl, C₆ to C₁₂ aryl, and C₃ to C₁₂ heteroaryl;
each R² is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkoxy, and C₁ to C₂₀ alkylamino;
Y is independently selected from a C₁ to C₂₅₀ hydrocarbyl group;
Y¹ is independently selected from hydrogen or a C₁ to C₂₅₀ hydrocarbyl group;
Z is independently selected from a C₂ to C₂₅₀ hydrocarbyl group comprising at least one primary or secondary amine;
Z¹ is independently selected from hydrogen or a C₁ to C₂₅₀ hydrocarbyl group;
n is independently 1, 2 or 3;
m is independently 1 or 2;
x is independently 0 to 3;
wherein n + m + x = 2 to 5; and
wherein the term hydrocarbyl refers to a monovalent group comprising a major proportion (i.e., more than 50 %) of hydrogen and carbon atoms, preferably consisting exclusively of hydrogen and carbon atoms.

2. The compound of Claim 1, wherein the compound has one of the following formulae:
i) wherein the compound has the formula (2a): 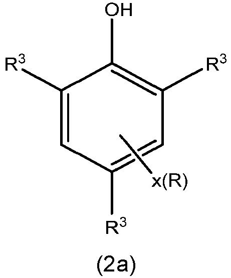 wherein:
each R³ is independently either an amine substituent of formula (6) or an amide substituent of formula (7), and at least one R³ is the amine substituent of formula (6) and at least one R³ is the amide substituent of formula (7): 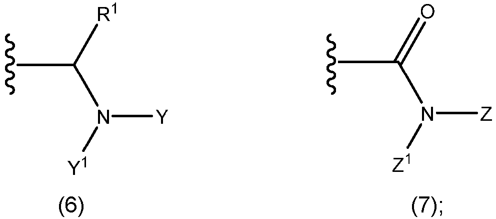
R, R¹, Y, Y¹, Z, and Z¹ are each as defined in Claim 1; and
x is independently 0 to 2:
preferably wherein the compound of formula (2a) has the formula (2a-i) or (2a-ii): 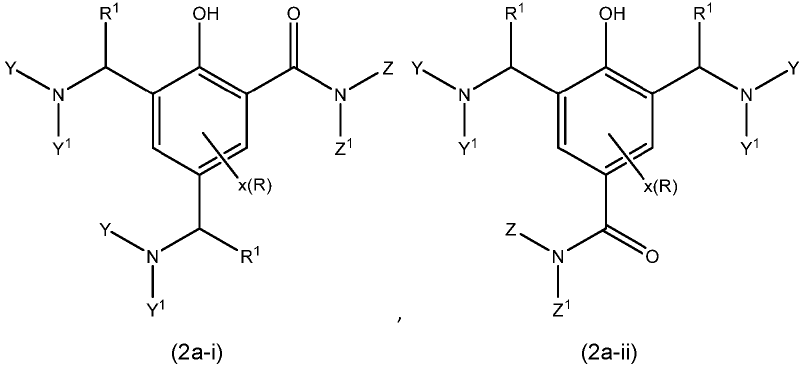 wherein:
R, R¹, Y, Y¹, Z and Z¹ are each as defined in Claim 1; and
x is independently 0 to 2:
or
ii) wherein the compound has the formula (2b): 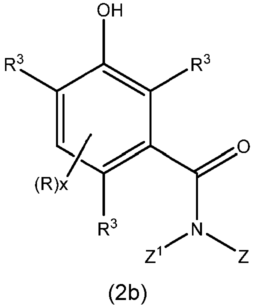 wherein:
each R³ is independently either an amine substituent of formula (6) or an amide substituent of formula (7), and at least one R³ is the amine substituent of formula (6); 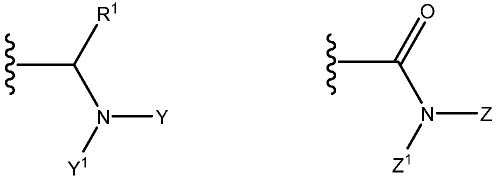 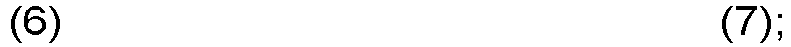
R, R¹, Y, Y¹, Z, and Z¹ are each as defined in Claim 1; and
x is independently 0 or 1.

3. A polymeric or co-polymeric compound comprising a repeating unit of formula (4): 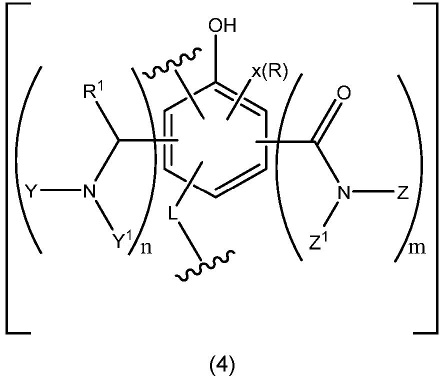 wherein:
R is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkyloxy, C₁ to C₂₀ alkylamino, -OH, -OR², -NH₂, -NHR², -NR²₂, -C(O)OH, -C(O)OR², -C(O)NH₂, -C(O)NHR², -C(O)NR²₂, -O(CO)H, -O(CO)R², -NH(CO)H, -NH(CO)R², -NR²(CO)H, -NR²(CO)R², -SH, -SR², -SO₂H, -SO₂R², -SO₃R², -SO₃H, -SiR²₃, -NO₂, -CN, -F, -Cl, -Br, and -I;
each R¹ group is independently selected from: -H, C₁ to C₁₀ alkyl, C₁ to C₁₀ haloalkyl, C₃ to C₁₂ cycloalkyl, C₂ to C₁₀ alkenyl, C₂ to C₁₀ alkynyl, C₆ to C₁₂ aryl, and C₃ to C₁₂ heteroaryl;
each R² is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkoxy, and C₁ to C₂₀ alkylamino;
Y is independently selected from a C₁ to C₂₅₀ hydrocarbyl group;
Y¹ is independently selected from hydrogen or a C₁ to C₂₅₀ hydrocarbyl group;
Z is independently selected from a C₂ to C₂₅₀ hydrocarbyl group comprising at least one primary or secondary amine;
Z¹ is independently either hydrogen or a C₁ to C₂₅₀ hydrocarbyl group; and
L is a linking group which connects the repeating unit to a single adjacent repeating unit of the polymer or co-polymer, and is selected from -CR⁹R¹⁰-;
each 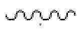 represents the attachment point between the linking group, L, of one repeating unit and a single adjacent repeating unit;
each R⁹ group is independently selected from: -H, C₁ to C₁₀ alkyl, C₁ to C₁₀ haloalkyl, C₃ to C₁₂ cycloalkyl, C₂ to C₁₀ alkenyl, C₂ to C₁₀ alkynyl, C₆ to C₁₂ aryl, C₃ to C₁₂ heteroaryl, and -C(O)H;
each R¹⁰ group is independently selected from: -H, C₁ to C₁₀ alkyl, C₁ to C₁₀ haloalkyl, C₃ to C₁₂ cycloalkyl, C₂ to C₁₀ alkenyl, C₂ to C₁₀ alkynyl, C₆ to C₁₂ aryl, and C₃ to C₁₂ heteroaryl;
or R⁹ and R¹⁰ are taken together to form a C₃ to C₁₂ cycloalkyl;
n is independently 1 or 2;
m is independently 1 or 2;
x is independently 0 or 1;
with the proviso that n + m + x = 2 or 3; and
wherein the term hydrocarbyl refers to a monovalent group comprising a major proportion (i.e., more than 50 %) of hydrogen and carbon atoms, preferably consisting exclusively of hydrogen and carbon atoms.

4. The polymeric or co-polymeric compound of Claim 3 comprising a repeating unit of formula (4a) and/or (4b): 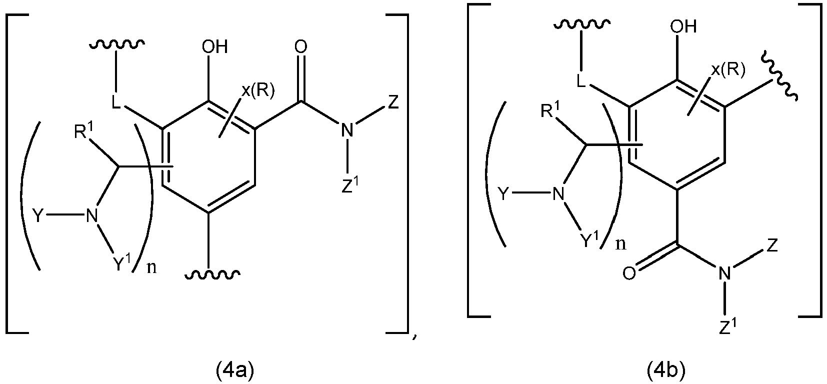 wherein:
L, 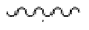 , R, R¹, R², Y, Y¹, Z, and Z¹ are as defined in Claim 3.
n is independently 1 or 2;
x is independently 0 or 1; and
with the proviso that n + x = 1 or 2.

5. A polymeric or co-polymeric compound comprising a repeating unit of formula (5): 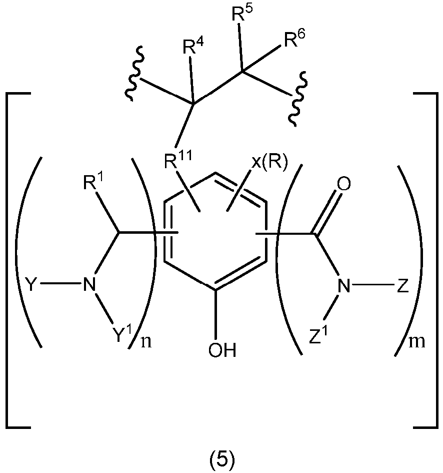 wherein:
R is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkyloxy, C₁ to C₂₀ alkylamino, -OH, -OR², -NH₂, -NHR², -NR²₂, - C(O)OH, -C(O)OR², -C(O)NH₂, -C(O)NHR², -C(O)NR²₂, -O(CO)H, -O(CO)R², - NH(CO)H, -NH(CO)R², -NR²(CO)H, -NR²(CO)R², -SH, -SR², -SO₂H, -SO₂R², - SO₃R², -SO₃H, -SiR²₃, -NO₂, -CN, -F, -Cl, -Br, and -I;
each R¹ group is independently selected from: -H, C₁ to C₁₀ alkyl, C₁ to C₁₀ haloalkyl, C₃ to C₁₂ cycloalkyl, C₂ to C₁₀ alkenyl, C₂ to C₁₀ alkynyl, C₆ to C₁₂ aryl, and C₃ to C₁₂ heteroaryl;
each R² is independently selected from: C₁ to C₂₀ alkyl, C₁ to C₂₀ haloalkyl, C₃ to C₂₀ cycloalkyl, C₂ to C₂₀ alkenyl, C₂ to C₂₀ alkynyl, C₁ to C₂₀ alkyl-C₃ to C₁₀ cycloalkyl, C₁ to C₂₀ alkoxy, and C₁ to C₂₀ alkylamino;
Y is independently a C₁ to C₂₅₀ hydrocarbyl group;
Y¹ is independently either hydrogen or a C₁ to C₂₅₀ hydrocarbyl group;
Z is independently a C₂ to C₂₅₀ hydrocarbyl group comprising at least one primary or secondary amine;
Z¹ is independently either hydrogen or a C₁ to C₂₅₀ hydrocarbyl group; and R⁴, R⁵ and R⁶ are independently selected from: -H, C₁ to C₁₀ alkyl, -F, -Cl, -Br, -I, -OH, -OR¹, -CN, -C(O)OH, -C(O)OR¹, -C(O)NH₂, -C(O)NHR¹, -C(O)NR¹₂, -O(CO)H, or -O(CO)R¹;
each 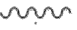 represents an attachment point between one repeating unit and a single adjacent repeating unit;
R¹¹ is either a direct bond or -OC(O)-;
n is independently 1, 2 or 3;
m is independently 1, 2 or 3;
x is independently 0 to 2;
with the proviso that n + m + x = 2 to 4;
wherein the term hydrocarbyl refers to a monovalent group comprising a major proportion (i.e., more than 50 %) of hydrogen and carbon atoms, preferably consisting exclusively of hydrogen and carbon atoms.

6. The polymeric or co-polymeric compound of Claim 5 comprising a repeating unit of formula (5) has the formula (5a): 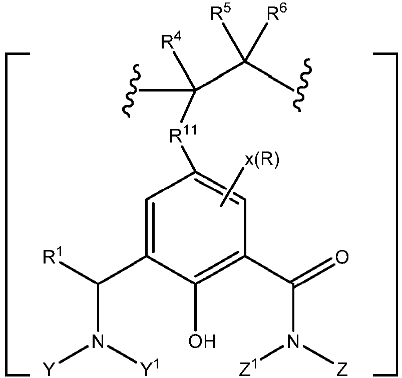 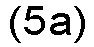 wherein: 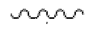 , R, R¹, R⁴, R⁵, R⁶, Y, Y¹, Z, Z¹ and R¹¹ are as defined in Claim 5; and x is independently 0, 1 or 2.

7. The compound of any preceding claim wherein one or more of the following is satisfied:
I) at least one Y group comprises an amine, preferably wherein each Y group comprises an amine; preferably wherein at least one Y group comprises a primary or secondary amine, more preferably wherein each Y group comprises a primary or secondary amine;
II) each Y and/or each Z are independently selected from a group of formula - (R⁷-NH-)_{q}-(CO)-R⁸-(CO)NH-(R⁷-NH-)_{q}-H;
wherein:
each R⁸ is independently a C₆ to C₇₀ branched or straight chain alkyl, cycloalkyl, alkenyl, or cycloalkenyl group, preferably wherein each R⁸ is independently a C₃₀ to C₃₆ branched chain alkyl, cycloalkyl, alkenyl, or cycloalkenyl group; and
each -(R⁷-NH-)q moiety independently satisfies one of the following conditions i) to iii):
i) R⁷ = a C₂ to C₁₂ alkyl group; and q = 1 to 7;
ii) R⁷ = a C₃ to C₁₈ cycloalkyl group, and q = 1 to 3; or
iii) R⁷ = a C₆ to C₁₈ aromatic or alkylaromatic group, and q = 1 to 3;
III) each Y and/or each Z are independently either:
a) a hydrocarbyl chain having the formula: -(R⁷-NH-)_{q}-H, wherein each -(R⁷-NH-)_{q}-H group independently satisfies one of the following conditions i) to iii):
i) R⁷ = a C₂ to C₁₂ alkyl group, and q = 1 to 7;
ii) R⁷ = a C₃ to C₁₈ cycloalkyl group, and q = 1 to 3; or
iii) R⁷ = a C₆ to C₁₈ aromatic or alkylaromatic group, and q = 1 to 3; or
b) a hydrocarbyl chain having the formula: -(R⁷)q-CH₂CH(CH₃)NH₂, wherein each -(R⁷)_{q}-CH₂CH(CH₃)NH₂ group independently satisfies one of the following conditions:
i) R⁷ = a C₂ to C₃ ether group, and q = 1 to 80; or
ii) R⁷ = a C₄ to C₈ ether group, and q = 1 to 30:
IV) each Y' and/or each Z¹ are independently selected from -H, -Me, -Et, - CH₂CH₂NH₂, -CH₂CH₂NHCH₂CH₂NH₂, and -(R⁷-NH-)_{q}-H, wherein R⁷ is a C₂ to C₁₂ alkyl group, and q = 1 to 3; preferably wherein each Y' and/or each Z¹ are -H;
V) each R group is independently selected from: C₁ to C₂₀ alkyl, C₃ to C₂₀ cycloalkyl, C₂ to C₂₀ alkenyl, C₂ to C₂₀ alkynyl, C₁ to C₂₀ alkyloxy, C₁ to C₂₀ alkylamino, -OH, -OR², -NH₂, -NHR², -NR²₂, -C(O)NH₂, -C(O)NHR², - C(O)NR²₂, -O(CO)H, -O(CO)R⁴, -NH(CO)H, -NH(CO)R², -NR²(CO)H, - NR⁴(CO)R², -SH, -SR², -SiR²₃, -F, -Cl, -Br, and -I; and wherein each R² is independently selected from: C₁ to C₂₀ alkyl, C₃ to C₂₀ cycloalkyl, C₂ to C₂₀ alkenyl, C₂ to C₂₀ alkynyl, C₁ to C₂₀ alkoxy, and C₁ to C₂₀ alkylamino; preferably wherein each R group is independently selected from: C₁ to C₁₀ alkyl, C₃ to C₁₀ cycloalkyl, C₂ to C₁₀ alkenyl, C₂ to C₁₀ alkynyl, C₁ to C₁₀ alkyloxy, C₁ to C₁₀ alkylamino, -OH, -OR², -NH₂, -NHR², and -NR²₂; and wherein each R² is independently selected from: C₁ to C₁₀ alkyl, C₃ to C₁₀ cycloalkyl, C₂ to C₁₀ alkenyl, C₂ to C₁₀ alkynyl, C₁ to C₁₀ alkoxy, and C₁ to C₁₀ alkylamino; more preferably wherein R is -OH; and/or
VI) wherein R¹ is furan.

8. A method of preparing an aromatic or heteroaromatic amide Mannich base for use as an epoxy resin curative and/or accelerator, said method comprising:
i) reacting an optionally substituted aromatic or heteroaromatic acid, or ester thereof, selected from hydroxy benzoic acid, hydroxypyridine carboxylic acid, pyrrole carboxylic acid, thiophene carboxylic acid, and furan carboxylic acid or an ester thereof, with a first polyamine having at least two nucleophilic amines, to form an aromatic or heteroaromatic amide;
ii) performing a Mannich reaction using the aromatic or heteroaromatic amide, an aldehyde, and a second amine having at least one nucleophilic amine.

9. A method of preparing a polymeric hydroxy benzamide Mannich base for use as an epoxy resin curative and/or accelerator, said method comprising:
i) performing a polycondensation reaction with an optionally substituted hydroxy benzoic acid, or ester thereof, and a first aldehyde, ketone or hexamine to give a polymeric hydroxy benzoic acid, or ester thereof;
ii) reacting the polymeric hydroxy benzoic acid, or ester thereof, with a first polyamine having at least two nucleophilic amines, to give a polymeric hydroxy benzamide;
iii) performing a Mannich reaction using the polymeric hydroxy benzamide, a second aldehyde, and a second amine having at least one nucleophilic amine.

10. A method of preparing a polymeric hydroxy benzamide Mannich base for use as an epoxy resin curative and/or accelerator, said method comprising:
i) performing a vinyl polymerisation reaction with optionally substituted and alkenyl substituted hydroxy benzoic acid, or ester thereof, to give a polymeric hydroxy benzoic acid, or ester thereof;
ii) reacting the polymeric hydroxy benzoic acid, or ester thereof, with a first polyamine having at least two nucleophilic amines, to give a polymeric hydroxy benzamide;
iii) performing a Mannich reaction using the polymeric hydroxy benzamide, an aldehyde, and a second amine having at least one nucleophilic amine.

11. The method of any one of Claims 8 to 10 wherein the second amine is a polyamine having at least one nucleophilic amine, preferably wherein the second amine is a polyamine having at least two nucleophilic amines.

12. An epoxy resin curative composition comprising a compound as defined in any one of Claims 1 to 7.

13. A method for preparing a cured epoxy resin, said method comprising:
a) contacting an epoxy resin with a compound as defined in any one of Claims 1 to 7; and
b) forming a cured epoxy resin, preferably wherein the epoxy resin is selected from glycidyl amines, epoxidized novolacs and bisphenols (A or F) or halogenated analogues thereof.

14. A cured epoxy resin prepared, or preparable, by the method of Claim 13.

15. Use of a compound as defined in any one of Claims 1 to 7 for causing the crosslinking of an epoxy resin.

## Patentansprüche

1. Eine Verbindung der Formel (2): 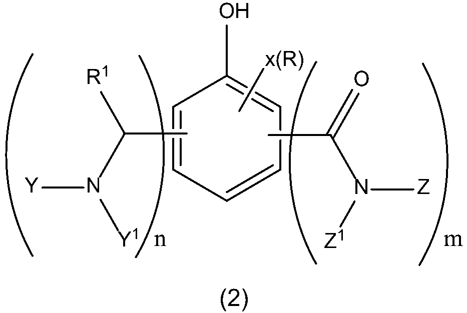
wobei:
jede R-Gruppe unabhängig aus den Folgenden ausgewählt ist: C₁- bis C₂₀-Alkyl, C₁- bis C₂₀-Halogenalkyl, C₃- bis C₂₀-Cycloalkyl, C₂- bis C₂₀-Alkenyl, C₂- bis C₂₀-Alkinyl, C₁- bis C₂₀-Alkyl-C₃- bis C₁₀-Cycloalkyl, C₁- bis C₂₀-Alkyloxy, C₁- bis C₂₀-Alkylamino, -OH, -OR², -NH₂, -NHR², -NR²₂, -C(O)OH, -C(O)OR², -C(O)NH₂, - C(O)NHR², -C(O)NR²₂, -O(CO)H, -O(CO)R², -NH(CO)H, -NH(CO)R², -NR²(CO)H, - NR²(CO)R², -SH, -SR², -SO₂H, -SO₂R², -SO₃R², -SO₃H, -SiR²₃, -NO₂, -CN, -F, -Cl, - Br und -I;
jede R¹-Gruppe unabhängig aus den Folgenden ausgewählt ist: -H, C₁- bis C₁₀-Alkyl, C₁- bis C₁₀-Halogenalkyl, C₃- bis C₁₂-Cycloalkyl, C₂- bis C₁₀-Alkenyl, C₂- bis C₁₀-Alkinyl, C₆- bis C₁₂-Aryl und C₃- bis C₁₂-Heteroaryl;
jedes R² unabhängig aus den Folgenden ausgewählt ist: C₁- bis C₂₀-Alkyl, C₁-bis C₂₀-Halogenalkyl, C₃- bis C₂₀-Cycloalkyl, C₂- bis C₂₀-Alkenyl, C₂- bis C₂₀-Alkinyl, C₁- bis C₂₀-Alkyl-C₃- bis C₁₀-Cycloalkyl, C₁- bis C₂₀-Alkoxy und C₁- bis C₂₀-Alkylamino;
Y unabhängig aus einer C₁- bis C₂₅₀-Hydrocarbylgruppe ausgewählt ist;
Y¹ unabhängig aus Wasserstoff oder einer C₁- bis C₂₅₀-Hydrocarbylgruppe ausgewählt ist;
Z unabhängig aus einer C₂- bis C₂₅₀-Hydrocarbylgruppe ausgewählt ist, die mindestens ein primäres oder sekundäres Amin umfasst;
Z¹ unabhängig aus Wasserstoff oder einer C₁- bis C₂₅₀-Hydrocarbylgruppe ausgewählt ist;
n unabhängig 1, 2 oder 3 ist;
m unabhängig 1 oder 2 ist;
x unabhängig 0 bis 3 ist;
wobei n + m + x = 2 bis 5; und
wobei sich der Begriff Hydrocarbyl auf eine einwertige Gruppe bezieht, die zu einem größeren Teil (d. h. zu mehr als 50 %) Wasserstoff- und Kohlenstoffatome umfasst, die bevorzugt ausschließlich aus Wasserstoff- und Kohlenstoffatomen besteht.

2. Verbindung nach Anspruch 1, wobei die Verbindung eine der folgenden Formeln aufweist:
i) wobei die Verbindung die Formel (2a) aufweist: 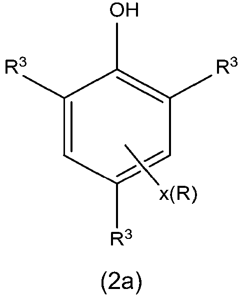 wobei:
jedes R³ unabhängig entweder ein Aminsubstituent der Formel (6) oder ein Amidsubstituent der Formel (7) ist und mindestens ein R³ der Aminsubstituent der Formel (6) ist und mindestens ein R³ der Amidsubstituent der Formel (7) ist: 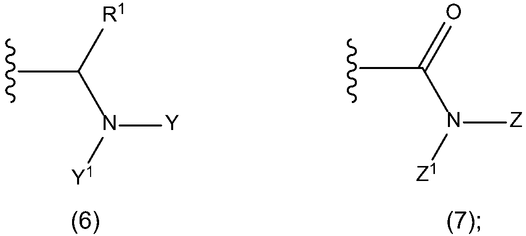
R, R¹, Y, Y¹, Z und Z¹ jeweils wie in Anspruch 1 definiert sind; und
x unabhängig 0 bis 2 ist:
wobei die Verbindung der Formel (2a) bevorzugt die Formel (2a-i) oder (2a-ii) aufweist: 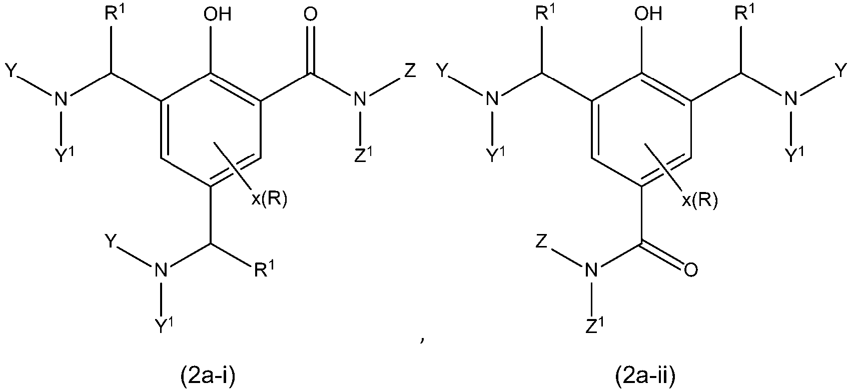 wobei:
R, R¹, Y, Y¹, Z und Z¹ jeweils wie in Anspruch 1 definiert sind; und
x unabhängig 0 bis 2 ist:
oder
ii) wobei die Verbindung die Formel (2b) aufweist: 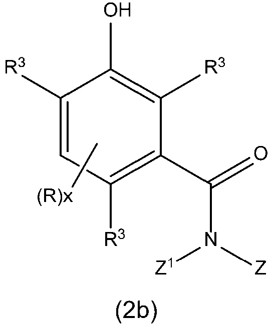 wobei:
jedes R³ unabhängig entweder ein Aminsubstituent der Formel (6) oder ein Amidsubstituent der Formel (7) ist und mindestens ein R³ der Aminsubstituent der Formel (6) ist; 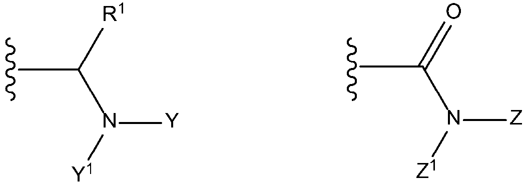 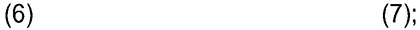
R, R¹, Y, Y¹, Z, und Z¹ jeweils wie in Anspruch 1 definiert sind; und
x unabhängig 0 oder 1 ist.

3. Eine polymere oder copolymere Verbindung, die eine Repetiereinheit der Formel (4) umfasst: 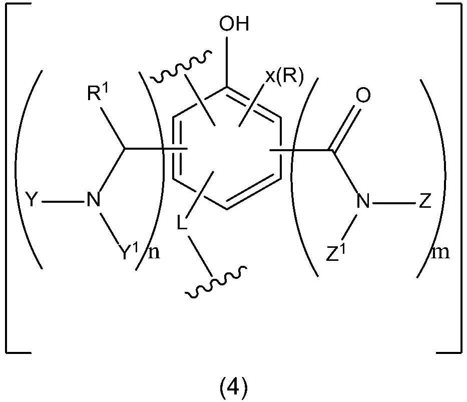 wobei:
R unabhängig aus den Folgenden ausgewählt ist: C₁- bis C₂₀-Alkyl, C₁- bis C₂₀-Halogenalkyl, C₃- bis C₂₀-Cycloalkyl, C₂- bis C₂₀-Alkenyl, C₂- bis C₂₀-Alkinyl, C₁- bis C₂₀-Alkyl-C₃- bis C₁₀-Cycloalkyl, C₁- bis C₂₀-Alkyloxy, C₁- bis C₂₀-Alkylamino, -OH, - OR², -NH₂, -NHR², -NR²₂, -C(O)OH, -C(O)OR², -C(O)NH₂, -C(O)NHR², -C(O)NR²₂, - O(CO)H, -O(CO)R², -NH(CO)H, -NH(CO)R², -NR²(CO)H, -NR²(CO)R², -SH, - SR², -SO₂H, -SO₂R², -SO₃R², -SO₃H, -SiR²₃, -NO₂, -CN, -F, -Cl, -Br und -I;
jede R¹-Gruppe unabhängig aus den Folgenden ausgewählt ist: -H, C₁- bis C₁₀-Alkyl, C₁- bis C₁₀-Halogenalkyl, C₃- bis C₁₂-Cycloalkyl, C₂- bis C₁₀-Alkenyl, C₂- bis C₁₀-Alkinyl, C₆- bis C₁₂-Aryl und C₃- bis C₁₂-Heteroaryl;
jedes R² unabhängig aus den Folgenden ausgewählt ist: C₁- bis C₂₀-Alkyl, C₁-bis C₂₀-Halogenalkyl, C₃- bis C₂₀-Cycloalkyl, C₂- bis C₂₀-Alkenyl, C₂- bis C₂₀-Alkinyl, C₁- bis C₂₀-Alkyl-C₃- bis C₁₀-Cycloalkyl, C₁- bis C₂₀-Alkoxy und C₁- bis C₂₀-Alkylamino;
Y unabhängig aus einer C₁- bis C₂₅₀-Hydrocarbylgruppe ausgewählt ist;
Y¹ unabhängig aus Wasserstoff oder einer C₁- bis C₂₅₀-Hydrocarbylgruppe ausgewählt ist;
Z unabhängig aus einer C₂- bis C₂₅₀-Hydrocarbylgruppe ausgewählt ist, die mindestens ein primäres oder sekundäres Amin umfasst;
Z¹ unabhängig entweder Wasserstoff oder eine C₁- bis C₂₅₀-Hydrocarbylgruppe ist; und
L eine Verknüpfungsgruppe ist, die die Repetiereinheit mit einer einzelnen benachbarten Repetiereinheit des Polymers oder Copolymers verbindet, und aus - CR⁹R¹⁰- ausgewählt ist;
jedes 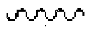 den Verbindungspunkt zwischen der Verknüpfungsgruppe, L, einer Repetiereinheit und einer einzelnen benachbarten Repetiereinheit darstellt;
jede R⁹-Gruppe unabhängig aus den Folgenden ausgewählt ist: -H, C₁- bis C₁₀-Alkyl, C₁- bis C₁₀-Halogenalkyl, C₃- bis C₁₂-Cycloalkyl, C₂- bis C₁₀-Alkenyl, C₂- bis C₁₀-Alkinyl, C₆- bis C₁₂-Aryl, C₃- bis C₁₂-Heteroaryl und -C(O)H;
jede R¹⁰-Gruppe unabhängig aus den Folgenden ausgewählt ist: -H, C₁- bis C₁₀-Alkyl, C₁- bis C₁₀-Halogenalkyl, C₃- bis C₁₂-Cycloalkyl, C₂- bis C₁₀-Alkenyl, C₂- bis C₁₀-Alkinyl, C₆- bis C₁₂-Aryl und C₃- bis C₁₂-Heteroaryl;
oder R⁹ und R¹⁰ zusammengenommen werden, um ein C₃- bis C₁₂-Cycloalkyl zu bilden;
n unabhängig 1 oder 2 ist;
m unabhängig 1 oder 2 ist;
x unabhängig 0 oder 1 ist;
mit der Maßgabe, dass n + m + x = 2 oder 3; und
wobei sich der Begriff Hydrocarbyl auf eine einwertige Gruppe bezieht, die zu einem größeren Teil (d. h. zu mehr als 50 %) Wasserstoff- und Kohlenstoffatome umfasst, die bevorzugt ausschließlich aus Wasserstoff- und Kohlenstoffatomen besteht.

4. Polymere oder copolymere Verbindung nach Anspruch 3, die eine Repetiereinheit der Formel (4a) und/oder (4b) umfasst: 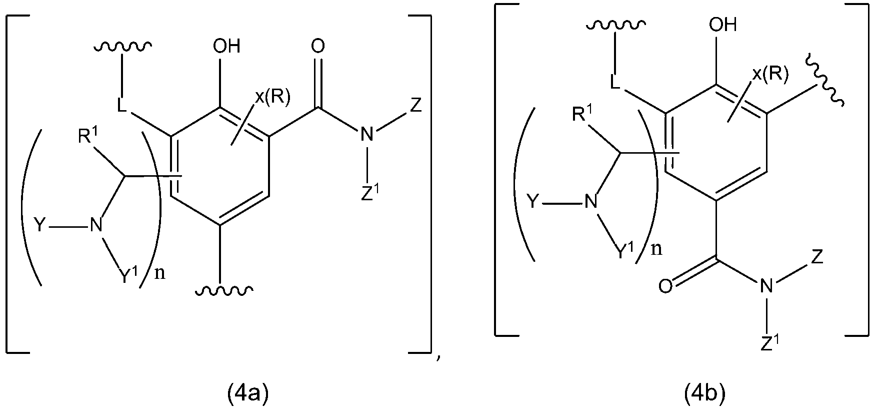 wobei:
L, 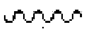 , R, R¹, R², Y, Y¹, Z und Z¹ wie in Anspruch 3 definiert sind.
n unabhängig 1 oder 2 ist;
x unabhängig 0 oder 1 ist; und
mit der Maßgabe, dass n + x = 1 oder 2.

5. Eine polymere oder copolymere Verbindung, die eine Repetiereinheit der Formel (5) umfasst: 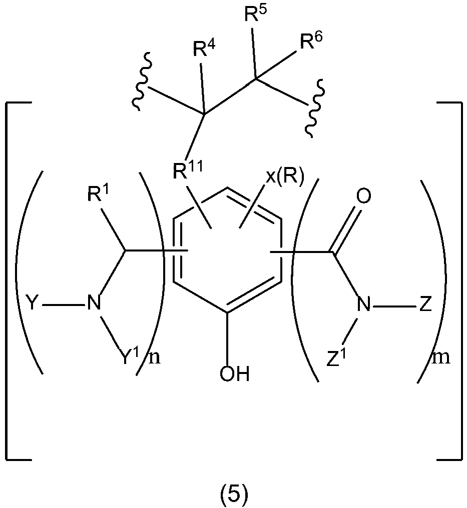 wobei:
R unabhängig aus den Folgenden ausgewählt ist: C₁- bis C₂₀-Alkyl, C₁- bis C₂₀-Halogenalkyl, C₃- bis C₂₀-Cycloalkyl, C₂- bis C₂₀-Alkenyl, C₂- bis C₂₀-Alkinyl, C₁-bis C₂₀-Alkyl-C₃- bis C₁₀-Cycloalkyl, C₁- bis C₂₀-Alkyloxy, C₁- bis C₂₀-Alkylamino, -OH, -OR², -NH₂, -NHR², -NR²₂, -C(O)OH, -C(O)OR², -C(O)NH₂, -C(O)NHR², -C(O)NR²₂, - O(CO)H, -O(CO)R², -NH(CO)H, -NH(CO)R², -NR²(CO)H, -NR²(CO)R², -SH, -SR², - SO₂H, -SO₂R², -SO₃R², -SO₃H, -SiR²₃, -NO₂, -CN, -F, -Cl, -Br und -I;
jede R¹-Gruppe unabhängig aus den Folgenden ausgewählt ist: -H, C₁- bis C₁₀-Alkyl, C₁- bis C₁₀-Halogenalkyl, C₃- bis C₁₂-Cycloalkyl, C₂- bis C₁₀-Alkenyl, C₂- bis C₁₀-Alkinyl, C₆- bis C₁₂-Aryl und C₃- bis C₁₂-Heteroaryl;
jedes R² unabhängig aus den Folgenden ausgewählt ist: C₁- bis C₂₀-Alkyl, C₁-bis C₂₀-Halogenalkyl, C₃- bis C₂₀-Cycloalkyl, C₂- bis C₂₀-Alkenyl, C₂- bis C₂₀-Alkinyl, C₁- bis C₂₀-Alkyl-C₃- bis C₁₀-Cycloalkyl, C₁- bis C₂₀-Alkoxy und C₁- bis C₂₀-Alkylamino;
Y unabhängig eine C₁- bis C₂₅₀-Hydrocarbylgruppe ist;
Y¹ unabhängig entweder Wasserstoff oder eine C₁- bis C₂₅₀-Hydrocarbylgruppe ist;
Z unabhängig eine C₂- bis C₂₅₀-Hydrocarbylgruppe ist, die mindestens ein primäres oder sekundäres Amin umfasst;
Z¹ unabhängig entweder Wasserstoff oder eine C₁- bis C₂₅₀-Hydrocarbylgruppe ist; und
R⁴, R⁵ und R⁶ unabhängig aus den Folgenden ausgewählt sind: -H, C₁- bis C₁₀-Alkyl, -F, -Cl, -Br, -I, -OH, -OR¹, -CN, -C(O)OH, -C(O)OR¹, -C(O)NH₂, -C(O)NHR¹, - C(O)NR¹₂, -O(CO)H oder -O(CO)R¹;
jedes 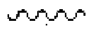 einen Verbindungspunkt zwischen einer Repetiereinheit und einer einzelnen angrenzenden Repetiereinheit darstellt;
R¹¹ entweder eine direkte Bindung oder -OC(O)- ist;
n unabhängig 1, 2 oder 3 ist;
m unabhängig 1, 2 oder 3 ist;
x unabhängig 0 bis 2 ist;
mit der Maßgabe, dass n + m + x = 2 bis 4;
wobei sich der Begriff Hydrocarbyl auf eine einwertige Gruppe bezieht, die zu einem größeren Teil (d. h. zu mehr als 50 %) Wasserstoff- und Kohlenstoffatome umfasst, die bevorzugt ausschließlich aus Wasserstoff- und Kohlenstoffatomen besteht.

6. Polymere oder copolymere Verbindung nach Anspruch 5, die eine Repetiereinheit der Formel (5) umfasst, die Formel (5a) aufweist: 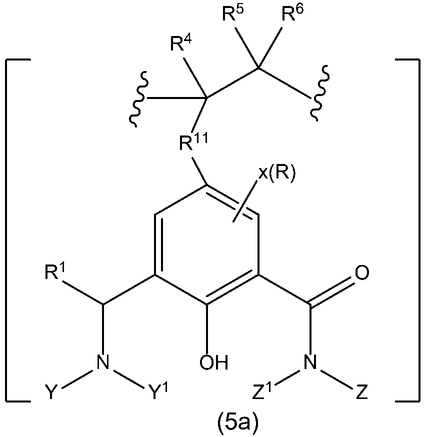 wobei:
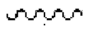 , R, R⁷, R⁴, R⁵, R⁶, Y, Y¹, Z, Z¹ und R¹¹ wie in Anspruch 5 definiert sind; und
x unabhängig 0, 1 oder 2 ist.

7. Verbindung nach einem der vorhergehenden Ansprüche, wobei eines oder mehrere der Folgenden erfüllt sind:
I) mindestens eine Y-Gruppe umfasst ein Amin, wobei jede Y-Gruppe bevorzugt ein Amin umfasst; wobei mindestens eine Y-Gruppe bevorzugt ein primäres oder sekundäres Amin umfasst, wobei jede Y-Gruppe bevorzugter ein primäres oder sekundäres Amin umfasst;
II) jedes Y und/oder jedes Z sind unabhängig aus einer Gruppe der Formel -(R⁷-NH-)_{q}-(CO)-R⁸-(CO)NH-(R⁷-NH-)_{q}-H ausgewählt;
wobei:
jedes R⁸ unabhängig eine verzweigt- oder geradkettige C₆- bis C₇₀-Alkyl-, - Cycloalkyl, -Alkenyl- oder -Cycloalkenylgruppe ist, wobei jedes R⁸ bevorzugt unabhängig eine verzweigtkettige C₃₀- bis C₃₆-Alkyl-, -Cycloalkyl-, -Alkenyl-oder -Cycloalkenylgruppe ist; und
jeder -(R⁷-NH-)_{q}-Teil unabhängig eine der folgenden Bedingungen i) bis iii) erfüllt:
i) R⁷ = eine C₂- bis C₁₂-Alkylgruppe; und q = 1 bis 7;
ii) R⁷ = eine C₃- bis C₁₈-Cycloalkylgruppe, und q = 1 bis 3; oder
iii) R⁷ = eine aromatische oder alkylaromatische C₆- bis C₁₈-Gruppe, und q = 1 bis 3;
III) jedes Y und/oder jedes Z sind unabhängig entweder:
a) eine Hydrocarbylkette mit der Formel: -(R⁷-NH-)_{q}-H, wobei jede -(R⁷-NH-)_{q}-H-Gruppe unabhängig eine der folgenden Bedingungen i) bis iii) erfüllt:
i) R⁷ = eine C₂- bis C₁₂-Alkylgruppe, und q = 1 bis 7;
ii) R⁷ = eine C₃- bis C₁₈-Cycloalkylgruppe, und q = 1 bis 3; oder
iii) R⁷ = eine aromatische oder alkylaromatische C₆- bis C₁₈-Gruppe, und q = 1 bis 3; oder
b) eine Hydrocarbylkette mit der Formel: -(R⁷)q-CH₂CH(CH₃)NH₂, wobei jede -(R⁷)_{q}-CH₂CH(CH₃)NH₂-Gruppe unabhängig eine der folgenden Bedingungen erfüllt:
i) R⁷ = eine C₂- bis C₃-Ethergruppe, und q = 1 bis 80; oder
ii) R⁷ = eine C₄- bis C₈-Ethergruppe, und q = 1 bis 30:
IV) jedes Y¹ und/oder jedes Z¹ sind unabhängig aus -H, -Me, -Et, -CH₂CH₂NH₂, - CH₂CH₂NHCH₂CH₂NH₂ und -(R⁷-NH-)_{q}-H ausgewählt, wobei R⁷ eine C₂- bis C₁₂-Alkylgruppe ist, und q = 1 bis 3; wobei jedes Y¹ und/oder jedes Z¹ bevorzugt -H ist;
V) jede R-Gruppe ist unabhängig ausgewählt aus: C₁- bis C₂₀-Alkyl, C₃- bis C₂₀-Cycloalkyl, C₂- bis C₂₀-Alkenyl, C₂- bis C₂₀-Alkinyl, C₁- bis C₂₀-Alkyloxy, C₁- bis C₂₀-Alkylamino, -OH, -OR², -NH₂, -NHR², -NR²₂, -C(O)NH₂, -C(O)NHR², - C(O)NR²₂, -O(CO)H, -O(CO)R⁴, -NH(CO)H, -NH(CO)R², -NR²(CO)H, - NR⁴(CO)R², -SH, -SR², -SiR²₃, -F, -Cl, -Br und -I; und wobei jedes R² unabhängig aus den Folgenden ausgewählt ist: C₁- bis C₂₀-Alkyl, C₃- bis C₂₀-Cycloalkyl, C₂- bis C₂₀-Alkenyl, C₂- bis C₂₀-Alkinyl, C₁- bis C₂₀-Alkoxy und C₁-bis C₂₀-Alkylamino;
wobei jede R-Gruppe bevorzugt unabhängig aus den Folgenden ausgewählt ist: C₁- bis C₁₀-Alkyl, C₃- bis C₁₀-Cycloalkyl, C₂- bis C₁₀-Alkenyl, C₂- bis C₁₀-Alkinyl, C₁- bis C₁₀-Alkyloxy, C₁- bis C₁₀-Alkylamino, -OH, -OR², -NH₂, -NHR² und -NR²₂; und wobei jedes R² unabhängig aus den Folgenden ausgewählt ist: C₁- bis C₁₀-Alkyl, C₃- bis C₁₀-Cycloalkyl, C₂- bis C₁₀-Alkenyl, C₂- bis C₁₀-Alkinyl, C₁- bis C₁₀-Alkoxy und C₁- bis C₁₀-Alkylamino; wobei R bevorzugter -OH ist; und/oder
VI) wobei R¹ Furan ist.

8. Ein Verfahren zur Herstellung einer aromatischen oder heteroaromatischen Amid-Mannich-Base zur Verwendung als ein Epoxidharz-Härter und/oder -Beschleuniger, wobei das genannte Verfahren Folgendes umfasst:
i) Zur-Reaktion-Bringen einer optional substituierten aromatischen oder heteroaromatischen Säure oder eines Esters davon, ausgewählt aus Hydroxybenzoesäure, Hydroxypyridincarbonsäure, Pyrrolcarbonsäure, Thiophencarbonsäure und Furancarbonsäure oder einem Ester davon, mit einem ersten Polyamin, das mindestens zwei nukleophile Amine aufweist, um ein aromatisches oder heteroaromatisches Amid zu bilden;
ii) Durchführen einer Mannich-Reaktion unter Verwendung des aromatischen oder heteroaromatischen Amids, eines Aldehyds und eines zweiten Amins, das mindestens ein nukleophiles Amin aufweist.

9. Ein Verfahren zur Herstellung einer polymeren Hydroxybenzamid-Mannich-Base zur Verwendung als ein Epoxidharz-Härter und/oder -Beschleuniger, wobei das genannte Verfahren Folgendes umfasst:
i) Durchführen einer Polykondensationsreaktion mit einer optional substituierten Hydroxybenzoesäure oder einem Ester davon und einem ersten Aldehyd, Keton oder Hexamin, um eine polymere Hydroxybenzoesäure oder ein Ester davon zu erhalten;
ii) Zur-Reaktion-Bringen der polymeren Hydroxybenzoesäure oder des Esters davon mit einem ersten Polyamin, das mindestens zwei nukleophile Amine aufweist, um ein polymeres Hydroxybenzamid zu erhalten;
iii) Durchführen einer Mannich-Reaktion unter Verwendung des polymeren Hydroxybenzamids, eines zweiten Aldehyds und eines zweiten Amins, das mindestens ein nukleophiles Amin aufweist.

10. Ein Verfahren zur Herstellung einer polymeren Hydroxybenzamid-Mannich-Base zur Verwendung als ein Epoxidharz-Härter und/oder -Beschleuniger, wobei das genannte Verfahren Folgendes umfasst:
i) Durchführen einer Vinylpolymerisationsreaktion mit optional substituierter und alkenylsubstituierter Hydroxybenzoesäure oder einem Ester davon, um eine polymere Hydroxybenzoesäure oder ein Ester davon zu erhalten;
ii) Zur-Reaktion-Bringen der polymeren Hydroxybenzoesäure oder des Esters davon mit einem ersten Polyamin, das mindestens zwei nukleophile Amine aufweist, um ein polymeres Hydroxybenzamid zu erhalten;
iii) Durchführen einer Mannich-Reaktion unter Verwendung des polymeren Hydroxybenzamids, eines Aldehyds und eines zweiten Amins, das mindestens ein nukleophiles Amin aufweist.

11. Verfahren nach einem der Ansprüche 8 bis 10, wobei das zweite Amin ein Polyamin ist, das mindestens ein nukleophiles Amin aufweist, wobei das zweite Amin bevorzugt ein Polyamin ist, das mindestens zwei nukleophile Amine aufweist.

12. Eine Epoxidharz-Härterzusammensetzung, die eine Verbindung wie in einem der Ansprüche 1 bis 7 definiert umfasst.

13. Ein Verfahren zur Herstellung eines gehärteten Epoxidharzes, wobei das genannte Verfahren Folgendes umfasst:
a) In-Kontakt-Bringen eines Epoxidharzes mit einer Verbindung wie in einem der Ansprüche 1 bis 7 definiert; und
b) Bilden eines gehärteten Epoxidharzes, wobei das Epoxidharz bevorzugt aus Glycidylaminen, epoxidierten Novolaken und Bisphenolen (A oder F) oder halogenierten Analoga davon ausgewählt ist.

14. Ein gehärtetes Epoxidharz, das durch das Verfahren nach Anspruch 13 hergestellt wurde oder hergestellt werden kann.

15. Verwendung einer Verbindung wie in einem der Ansprüche 1 bis 7 definiert zum Bewirken der Vernetzung eines Epoxidharzes.

## Revendications

1. Composé de formule (2) : 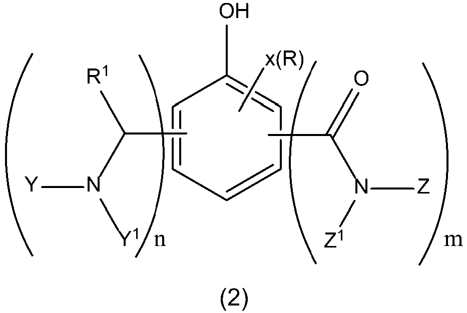 dans lequel :
chaque groupe R est indépendamment choisi parmi : un alkyle en C₁ à C₂₀, un haloalkyle en C₁ à C₂₀, un cycloalkyle en C₃ à C₂₀, un alcényle en C₂ à C₂₀, un alcynyle en C₂ à C₂₀, un alkyle en C₁ à C₂₀-cycloalkyle en C₃ à C₁₀, un alkyloxy en C₁ à C₂₀, un alkylamino en C₁ à C₂₀, -OH, -OR², -NH₂, -NHR², -NR²₂, -C(O)OH, -C(O)OR², - C(O)NH₂, -C(O)NHR², -C(O)NR²₂, -O(CO)H, -O(CO)R², -NH(CO)H, -NH(CO)R², - NR²(CO)H, -NR²(CO)R², -SH, -SR², -SO₂H, -SO₂R², -SO₃R², -SO₃H, -SiR²₃, -NO₂, - CN, -F, -Cl, -Br, et -I ;
chaque groupe R' est indépendamment choisi parmi : -H, un alkyle en C₁ à C₁₀, un haloalkyle en C₁ à C₁₀, un cycloalkyle en C₃ à C₁₂, un alcényle en C₂ à C₁₀, un alcynyle en C₂ à C₁₀, un aryle en C₆ à C₁₂, et un hétéroaryle en C₃ à C₁₂ ;
chaque R² est indépendamment choisi parmi : un alkyle en C₁ à C₂₀, un haloalkyle en C₁ à C₂₀, un cycloalkyle en C₃ à C₂₀, un alcényle en C₂ à C₂₀, un alcynyle en C₂ à C₂₀, un alkyle en C₁ à C₂₀-cycloalkyle en C₃ à C₁₀, un alcoxy en C₁ à C₂₀, et un alkylamino en C₁ à C₂₀ ;
Y est indépendamment choisi parmi un groupe hydrocarbyle en C₁ à C₂₅₀ ;
Y¹ est indépendamment choisi parmi l'hydrogène ou un groupe hydrocarbyle en C₁ à C₂₅₀ ;
Z est indépendamment choisi parmi un groupe hydrocarbyle en C₂ à C₂₅₀ comprenant au moins une amine primaire ou secondaire ;
Z¹ est indépendamment choisi parmi l'hydrogène ou un groupe hydrocarbyle en C₁ à C₂₅₀ ;
n est indépendamment 1, 2 ou 3 ;
m est indépendamment 1 ou 2 ;
x est indépendamment 0 à 3 ;
dans lequel n + m + x = 2 à 5 ; et
dans lequel le terme hydrocarbyle désigne un groupe monovalent comprenant une proportion majeure (c'est-à-dire plus de 50 %) d'atomes d'hydrogène et de carbone, de préférence constitué exclusivement d'atomes d'hydrogène et de carbone.

2. Composé selon la revendication 1, dans lequel le composé a l'une des formules suivantes :
i) dans lequel le composé a la formule (2a) : 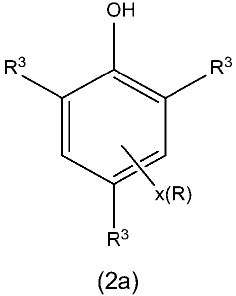 dans lequel :
chaque R³ est indépendamment soit un substituant amine de formule (6), soit un substituant amide de formule (7), et au moins un R³ est le substituant amine de formule (6) et au moins un R³ est le substituant amide de formule (7) : 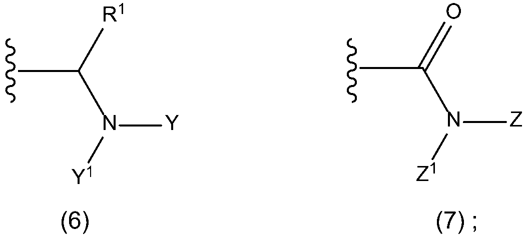
R, R¹, Y, Y¹, Z, et Z¹ sont chacun tels que définis dans la revendication 1 ; et
x est indépendamment 0 à 2 ;
de préférence dans lequel le composé de formule (2a) a la formule (2a-i) ou (2a-ii) : 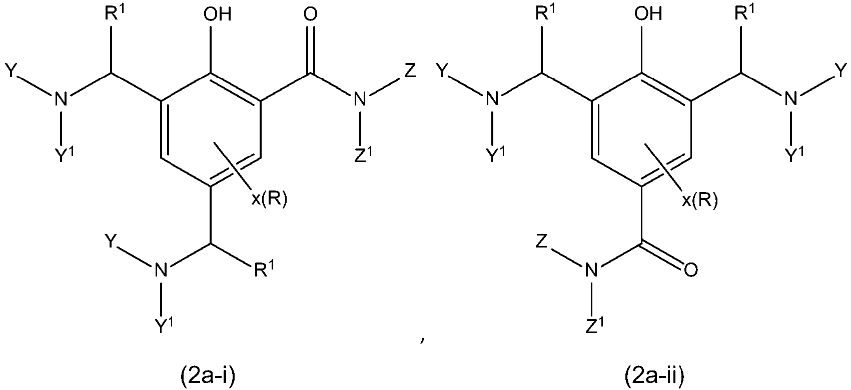 dans lequel :
R, R¹, Y, Y¹, Z et Z¹ sont chacun tels que définis dans la revendication 1 ; et
x est indépendamment 0 à 2 ;
ou
ii) dans lequel le composé a la formule (2b) : 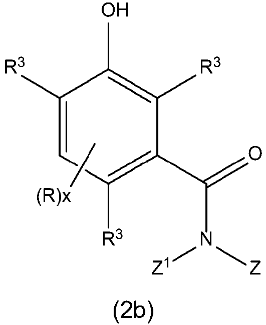 dans lequel :
chaque R³ est indépendamment soit un substituant amine de formule (6), soit un substituant amide de formule (7), et au moins un R³ est le substituant amine de formule (6) ; 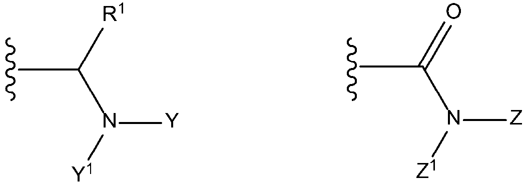 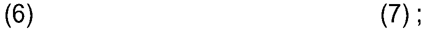
R, R¹, Y, Y¹, Z, et Z¹ sont chacun tels que définis dans la revendication 1 ; et
x est indépendamment 0 ou 1.

3. Composé polymérique ou copolymérique comprenant une unité de répétition de formule (4) : 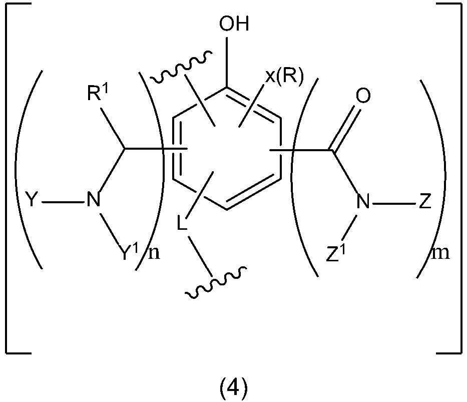 dans lequel :
R est indépendamment choisi parmi : un alkyle en C₁ à C₂₀, un haloalkyle en C₁ à C₂₀, un cycloalkyle en C₃ à C₂₀, un alcényle en C₂ à C₂₀, un alcynyle en C₂ à C₂₀, un alkyle en C₁ à C₂₀-cycloalkyle en C₃ à C₁₀, un alkyloxy en C₁ à C₂₀, un alkylamino en C₁ à C₂₀, -OH, -OR², -NH₂, -NHR², -NR²₂, -C(O)OH, -C(O)OR², -C(O)NH₂, - C(O)NHR², -C(O)NR²₂, -O(CO)H, -O(CO)R², -NH(CO)H, -NH(CO)R², -NR²(CO)H, - NR²(CO)R², -SH, -SR², -SO₂H, -SO₂R², -SO₃R², -SO₃H, -SiR²₃, -NO₂, -CN, -F, -CI, - Br, et -I ;
chaque groupe R¹ est indépendamment choisi parmi : -H, un alkyle en C₁ à C₁₀, un haloalkyle en C₁ à C₁₀, un cycloalkyle en C₃ à C₁₂, un alcényle en C₂ à C₁₀, un alcynyle en C₂ à C₁₀, un aryle en C₆ à C₁₂, et un hétéroaryle en C₃ à C₁₂ ;
chaque R² est indépendamment choisi parmi : un alkyle en C₁ à C₂₀, un haloalkyle en C₁ à C₂₀, un cycloalkyle en C₃ à C₂₀, un alcényle en C₂ à C₂₀, un alcynyle en C₂ à C₂₀, un alkyle en C₁ à C₂₀-cycloalkyle en C₃ à C₁₀, un alcoxy en C₁ à C₂₀, et un alkylamino en C₁ à C₂₀ ;
Y est indépendamment choisi parmi un groupe hydrocarbyle en C₁ à C₂₅₀ ;
Y¹ est indépendamment choisi parmi l'hydrogène ou un groupe hydrocarbyle en C₁ à C₂₅₀ ;
Z est indépendamment choisi parmi un groupe hydrocarbyle en C₂ à C₂₅₀ comprenant au moins une amine primaire ou secondaire ;
Z¹ est indépendamment soit de l'hydrogène ou un groupe hydrocarbyle en C₁ à C₂₅₀ ; et
L est un groupe de liaison qui connecte l'unité de répétition à une unité de répétition adjacente unique du polymère ou du copolymère, et est choisi parmi - CR⁹R¹⁰- ;
chaque 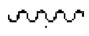 représente le point d'attache entre le groupe de liaison, L, d'une unité de répétition et une unité de répétition unique adjacente ;
chaque groupe R⁹ est indépendamment choisi parmi : -H, un alkyle en C₁ à C₁₀, un haloalkyle en C₁ à C₁₀, un cycloalkyle en C₃ à C₁₂, un alcényle en C₂ à C₁₀, un alcynyle en C₂ à C₁₀, un aryle en C₆ à C₁₂, et un hétéroaryle en C₃ à C₁₂, et -C(OH)H ;
chaque groupe R¹⁰ est indépendamment choisi parmi : -H, un alkyle en C₁ à C₁₀, un haloalkyle en C₁ à C₁₀, un cycloalkyle en C₃ à C₁₂, un alcényle en C₂ à C₁₀, un alcynyle en C₂ à C₁₀, un aryle en C₆ à C₁₂, et un hétéroaryle en C₃ à C₁₂ ;
ou R^{9 et} R¹⁰ sont pris ensemble pour former un cycloalkyle en C₃ à C₁₂ ;
n est indépendamment 1 ou 2 ;
m est indépendamment 1 ou 2 ;
x est indépendamment 0 ou 1 ;
à condition que n + m + x = 2 ou 3 ; et
dans lequel le terme hydrocarbyle désigne un groupe monovalent comprenant une proportion majeure (c'est-à-dire plus de 50 %) d'atomes d'hydrogène et de carbone, de préférence constitué exclusivement d'atomes d'hydrogène et de carbone.

4. Composé polymérique ou copolymérique selon la revendication 3 comprenant une unité de répétition de formule (4a) et/ou (4b) : 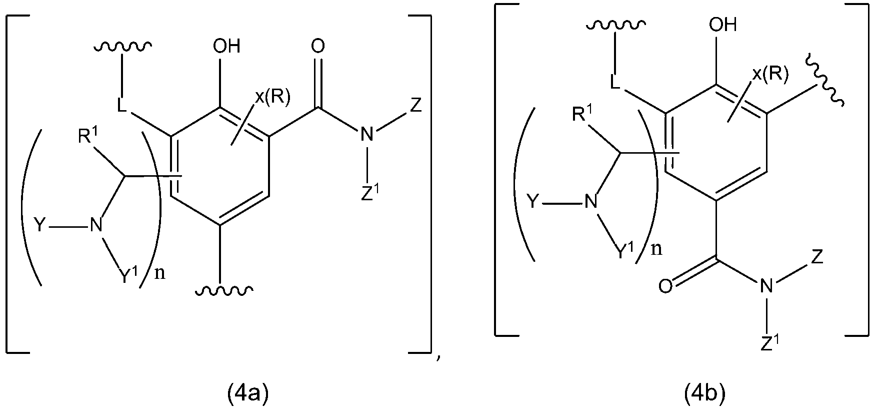 dans lequel :
L, 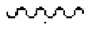 , R, R¹, R², Y, Y¹, Z, et Z¹ sont tels que définis dans la revendication 3.
n est indépendamment 1 ou 2 ;
x est indépendamment 0 ou 1 ; et
à condition que n + x = 1 ou 2.

5. Composé polymérique ou copolymérique comprenant une unité de répétition de formule (5) : 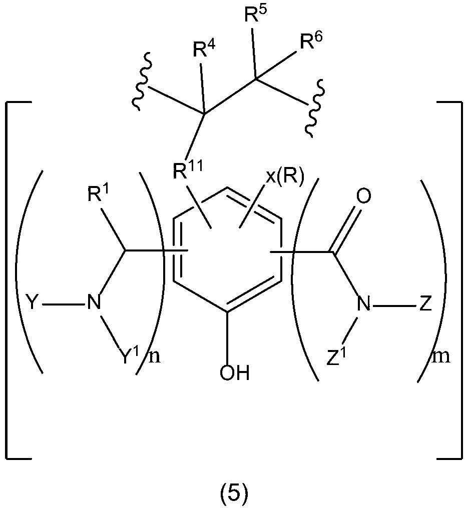 dans lequel :
R est indépendamment choisi parmi : un alkyle en C₁ à C₂₀, un haloalkyle en C₁ à C₂₀, un cycloalkyle en C₃ à C₂₀, un alcényle en C₂ à C₂₀, un alcynyle en C₂ à C₂₀, un alkyle en C₁ à C₂₀-un cycloalkyle en C₃ à C₁₀, un alkyloxy en C₁ à C₂₀, un alkylamino en C₁ à C₂₀, -OH, -OR², -NH₂, -NHR², -NR²₂, -C(O)OH, -C(O)OR², -C(O)NH₂, - C(O)NHR², -C(O)NR²₂, -O(CO)H, -O(CO)R², -NH(CO)H, -NH(CO)R², -NR²(CO)H, - NR²(CO)R², -SH, -SR², -SO₂H, -SO₂R², -SO₃R², -SO₃H, -SiR²₃, -NO₂, -CN, -F, -Cl, - Br, et -I;
chaque groupe R¹ est indépendamment choisi parmi : -H, un alkyle en C₁ à C₁₀, un haloalkyle en C₁ à C₁₀, un cycloalkyle en C₃ à C₁₂, un alcényle en C₂ à C₁₀, un alcynyle en C₂ à C₁₀, un aryle en C₆ à C₁₂, et un hétéroaryle en C₃ à C₁₂ ;
chaque R² est indépendamment choisi parmi : un alkyle en C₁ à C₂₀, un haloalkyle en C₁ à C₂₀, un cycloalkyle en C₃ à C₂₀, un alcényle en C₂ à C₂₀, un alcynyle en C₂ à C₂₀, un alkyle en C₁ à C₂₀-cycloalkyle en C₃ à C₁₀, un alcoxy en C₁ à C₂₀, et un alkylamino en C₁ à C₂₀ ;
Y est indépendamment un groupe hydrocarbyle en C₁ à C₂₅₀ ;
Y¹ est indépendamment soit de l'hydrogène ou un groupe hydrocarbyle en C₁ à C250 ;
Z est indépendamment un groupe hydrocarbyle en C₂ à C₂₅₀ comprenant au moins une amine primaire ou secondaire ;
Z¹ est indépendamment soit de l'hydrogène ou un groupe hydrocarbyle en C₁ à C₂₅₀ ; et
R⁴, R⁵ et R⁶ sont indépendamment choisis parmi : -H, un alkyle en C₁ à C₁₀, -F, -CI, -Br, -I, -OH, -OR¹, -CN, -C(O)OH, -C(O)OR¹, -C(O)NH₂, -C(O)NHR¹, -C(O)NR¹₂, - O(CO)H, ou -O(CO)R¹ ;
chaque 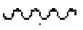 représente un point d'attache entre une unité de répétition et une unité de répétition unique adjacente ;
R¹¹ est soit une liaison directe soit -OC(O)- ;
n est indépendamment 1, 2 ou 3 ;
m est indépendamment 1, 2 ou 3 ;
x est indépendamment 0 à 2 ;
à condition que n + m + x = 2 à 4 ;
dans lequel le terme hydrocarbyle désigne un groupe monovalent comprenant une proportion majeure (c'est-à-dire plus de 50 %) d'atomes d'hydrogène et de carbone, de préférence constitué exclusivement d'atomes d'hydrogène et de carbone.

6. Composé polymère ou copolymère selon la revendication 5 comprenant une unité de répétition de formule (5) a la formule (5a) : 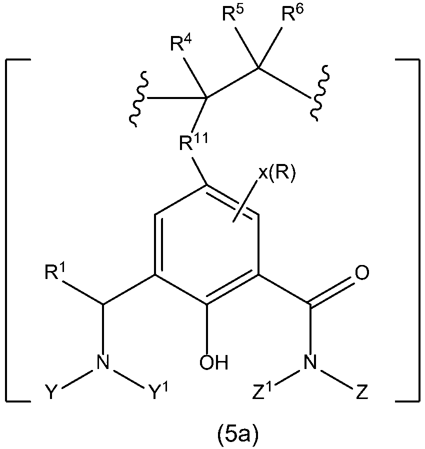 dans lequel :
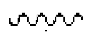 , R, R¹, R⁴, R⁵, R⁶, Y, Y¹, Z, Z¹ et R¹¹ sont tels que définis dans la revendication 5 ; et
x est indépendamment 0, 1 ou 2.

7. Composé selon l'une quelconque des revendications précédentes, dans lequel une ou plusieurs des conditions suivantes sont satisfaites :
I) au moins un groupe Y comprend une amine, de préférence dans lequel chaque groupe Y comprend une amine ; de préférence dans lequel au moins un groupe Y comprend une amine primaire ou secondaire, de manière plus préférée dans lequel chaque groupe Y comprend une amine primaire ou secondaire ;
II) chaque Y et/ou chaque Z sont indépendamment choisis dans un groupe de formule -(R⁷-NH-)_{q}-(CO)-R⁸-(CO)NH-(R⁷-NH-)_{q}-H ;
dans lequel :
chaque R⁸ est indépendamment un groupe alkyle, cycloalkyle, alcényle, ou cycloalcényle à chaîne ramifiée ou linéaire en C₆ à C₇₀, de préférence dans lequel chaque R⁸ est indépendamment un groupe alkyle, cycloalkyle, alcényle, ou cycloalcényle à chaîne ramifiée en C₃₀ à C₃₆ ; et
chaque groupement -(R⁷-NH-)_{q} répond indépendamment à l'une des conditions suivantes i) à iii) :
i) R⁷ = un groupe alkyle en C₂ à C₁₂ ; et q = 1 à 7 ;
ii) R⁷ = un groupe cycloalkyle en C₃ à C₁₈ , et q = 1 à 3 ; ou
iii) R⁷ = un groupe aromatique ou alkylaromatique en C₆ à C₁₈ , et q = 1 à 3 ;
III) chaque Y et/ou chaque Z sont indépendamment soit :
a) une chaîne hydrocarbyle ayant la formule : -(R⁷-NH-)_{q}-H, dans lequel chaque groupe -(R⁷-NH-)_{q}-H répond indépendamment à l'une des conditions suivantes i) à iii) :
i) R⁷ = un groupe alkyle en C₂ à C₁₂, et q = 1 à 7 ;
ii) R⁷ = un groupe cycloalkyle en C₃ à C₁₈, et q = 1 à 3 ; ou
iii) R⁷ = un groupe aromatique ou alkylaromatique en C₆ à C₁₈ , et q = 1 à 3 ; ou
b) une chaîne hydrocarbyle ayant la formule : -(R⁷)_{q}-CH₂CH(CH₃)NH₂, dans lequel chaque groupe -(R⁷)_{q}-CH₂CH(CH₃)NH₂ répond indépendamment à l'une des conditions suivantes :
i) R⁷ = un groupe éther en C₂ à C₃, et q = 1 à 80 ; ou
ii) R⁷ = un groupe éther en C₄ à C₈, et q = 1 à 30 :
IV) chaque Y¹ et/ou chaque Z¹ sont indépendamment choisis parmi -H, -Me, -Et, - CH₂CH₂NH₂, -CH₂CH₂NHCH₂CH₂NH₂ et -(R⁷-NH-)_{q}-H, dans lequel R⁷ est un groupe alkyle en C₂ à C₁₂, et q = 1 à 3 ; de préférence dans lequel chaque Y¹ et/ou chaque Z¹ sont -H ;
V) chaque groupe R est indépendamment choisi parmi : un alkyle en C₁ à C₂₀, un cycloalkyle en C₃ à C₂₀, un alcényle en C₂ à C₂₀, un alcynyle en C₂ à C₂₀, un alkyloxy en C₁ à C₂₀, un alkylamino en C₁ à C₂₀, -OH, -OR², -NH₂, -NHR², - NR²₂, -C(O)NH₂, -C(O)NHR², -C(O)NR²₂, -O(CO)H, -O(CO)R⁴, -NH(CO)H, - NH(CO)R², -NR²(CO)H, -NR⁴(CO)R², -SH, -SR², -SiR²₃, -F, -Cl, -Br et -I ; et dans lequel chaque R² est indépendamment choisi parmi : un alkyle en C₁ à C₂₀, un cycloalkyle en C₃ à C₂₀, un alcényle en C₂ à C₂₀, un alcynyle en C₂ à C₂₀, un alcoxy en C₁ à C₂₀ et un alkylamino en C₁ à C₂₀ ;
**de préférence,** chaque groupe R étant indépendamment choisi parmi : un alkyle en C₁ à C₁₀, un cycloalkyle en C₃ à C₁₀, un alcényle en C₂ à C₁₀, un alcynyle en C₂ à C₁₀, un alkyloxy en C₁ à C₁₀, un alkylamino en C₁ à C₁₀, -OH, -OR², -NH₂,-NHR² et -NR²₂ ; et dans lequel chaque R² est indépendamment choisi parmi : un alkyle en C₁ à C₁₀, un cycloalkyle en C₃ à C₁₀, un alcényle en C₂ à C₁₀, un alcynyle en C₂ à C₁₀, un alkyloxy en C₁ à C₁₀, et un alkylamino en C₁ à C₁₀ ; de manière plus préférée dans lequel R est -OH ; et/ou
VI) dans lequel R¹ est un furane.

8. Procédé de préparation d'une base de Mannich amide aromatique ou hétéroaromatique destinée à être utilisée comme un durcisseur et/ou accélérateur de résine époxyde, ledit procédé comprenant :
i) la réaction d'un acide aromatique ou hétéroaromatique facultativement substitué, ou d'un ester de celui-ci, choisi parmi l'acide hydroxybenzoïque, l'acide hydroxypyridine carboxylique, l'acide pyrrole carboxylique, l'acide thiophène carboxylique, et l'acide furane carboxylique ou un ester de celui-ci, avec une première polyamine ayant au moins deux amines nucléophiles, pour former un amide aromatique ou hétéroaromatique ;
ii) la réalisation d'une réaction de Mannich en utilisant l'amide aromatique ou hétéroaromatique, un aldéhyde, et une deuxième amine ayant au moins une amine nucléophile.

9. Procédé de préparation d'une base de Mannich d'hydroxybenzamide polymérique destinée à être utilisée comme un durcisseur et/ou accélérateur de résine époxy, ledit procédé comprenant :
i) la réalisation d'une réaction de polycondensation avec un acide hydroxybenzoïque facultativement substitué, ou un ester de celui-ci, et un premier aldéhyde, une première cétone ou une première hexamine pour donner un acide hydroxybenzoïque polymérique, ou un ester de celui-ci ;
ii) la réaction de l'acide hydroxybenzoïque polymérique, ou de l'ester de celui-ci, avec une première polyamine ayant au moins deux amines nucléophiles, pour donner un hydroxybenzamide polymérique ;
iii) la réalisation d'une réaction de Mannich en utilisant l'hydroxybenzamide polymérique, un deuxième aldéhyde, et une deuxième amine ayant au moins une amine nucléophilique.

10. Procédé de préparation d'une base de Mannich d'hydroxybenzamide polymérique destinée à être utilisée comme un durcisseur et/ou un accélérateur de résine époxy, ledit procédé comprenant :
i) la réalisation d'une réaction de polymérisation de vinyle avec un acide hydroxybenzoïque facultativement substitué et substitué avec alcényle, ou un ester de celui-ci, pour donner un acide hydroxybenzoïque polymérique, ou un ester de celui-ci ;
ii) la réaction de l'acide hydroxybenzoïque polymérique, ou de l'ester de celui-ci, avec une première polyamine ayant au moins deux amines nucléophiles, pour donner un hydroxybenzamide polymérique ;
iii) la réalisation d'une réaction de Mannich en utilisant l'hydroxybenzamide polymérique, un aldéhyde, et une deuxième amine ayant au moins une amine nucléophile.

11. Procédé selon l'une quelconque des revendications 8 à 10 dans lequel la deuxième amine est une polyamine ayant au moins une amine nucléophile, de préférence dans lequel la deuxième amine est une polyamine ayant au moins deux amines nucléophiles.

12. Composition de durcissement de résine époxy comprenant un composé tel que défini dans l'une quelconque des revendications 1 à 7.

13. Procédé destiné à préparer une résine époxy durcie, ledit procédé comprenant :
a) la mise en contact d'une résine époxy avec un composé tel que défini dans l'une quelconque des revendications 1 à 7 ; et
b) la formation d'une résine époxy durcie, de préférence dans laquelle la résine époxy est choisie parmi des glycidylamines, des novolacs époxydés et des bisphénols (A ou F) ou des analogues halogénés de ceux-ci.

14. Résine époxy durcie préparée, ou pouvant être préparée, par le procédé selon la revendication 13.

15. Utilisation d'un composé tel que défini selon l'une quelconque des revendications 1 à 7 destinée à provoquer la réticulation d'une résine époxy.
